# EUROPEAN PATENT APPLICATION

(11) **EP 4 342 899 A1**
(43) Date of publication of application: **27.03.2024**
(21) Application number: 22803978.0
(22) Date of filing: 17.05.2022
(51) Int. Cl.: C07D 473/18, C07D 519/00, A61K 31/522, A61P 35/00

(54) **SALT FORM AND CRYSTAL FORM OF HETEROCYCLIC SUBSTITUTED PURINONE DERIVATIVE**

(30) Priority: 19.05.2021 CN 202110547042; 27.04.2022 CN 202210459521
(71) Applicant: Zai Lab (Shanghai) Co., Ltd., Shanghai 201210 (CN)
(72) Inventor: CHEN, Kevin X, Shanghai 200131 (CN); XIA, Shanghua, Shanghai 200131 (CN); CHEN, Zhaoguo, Shanghai 200131 (CN); CHEN, Shuhui, Shanghai 200131 (CN)
(74) Representative: Murgitroyd & Company
(86) International application number: PCT/CN2022/093391
(87) International publication number: WO 2022/242658

(57) **Abstract**

Disclosed are a salt form and a crystal form of a heterocyclic substituted purinone derivative and a preparation method therefor. Specifically disclosed are a salt form and a crystal form of a compound of formula (I) and a preparation method therefor.

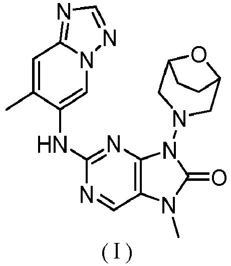

## Description

The present application claims the right of the following priorities:
CN202110547042.7, application date: May 19, 2021;
CN202210459521.8, application date: April 27, 2022.

### TECHNICAL FIELD

The present disclosure relates to a salt form and a crystal form of a heterocyclic substituted purinone derivative and a preparation method therefor.

### BACKGROUND

DNA breaks, especially double-strand breaks (DSBs), are extremely serious damages that can cause loss of genetic material and genetic recombination, thus leading to cancer or cell death. Eukaryotic cells have evolved a variety of mechanisms to cope with the serious threat caused by DNA double-strand breaks. This is called the DNA damage response (DDR) mechanism, which mainly includes the detection of DNA damage, signal transduction, and damage repair. DNA double-strand break repair mainly includes homologous recombination (HR) repair and non-homologous end joining (NHEJ) repair.

DNA-PK catalytic subunit (DNA-PKcs), a member of the phosphoinositide-3-kinase-related protein (PI3K-related kinase, PIKK) family, primarily targets the non-homologous end joining (NHEJ) repair of DNA double-strand breaks. It is an important member of DNA damage repair. During the repair of DNA double-strand breaks, the Ku70/Ku80 heterodimer specifically attaches to the site of the double-strand breaks through a preformed channel, recognizes the double-strand breaks and binds to the ends of the breaks, respectively. Then, in an ATP-dependent manner, the Ku70/Ku80 heterodimer slides a certain distance along the DNA chain towards both ends, forming a KU-DNA complex and recruiting DNA-PKcs to the site of the break for binding. Subsequently, the Ku dimer moves inward, activating DNA-PKcs and causing its self-phosphorylation. Finally, the phosphorylated DNA-PKcs guide damage signal transduction and recruit DNA end processing-related proteins such as PNKP, XRCC4, XLF, Pol X, and DNA ligase IV to participate in the completion of double-strand break repair.

Currently, the main mechanism by which DNA-damaging chemotherapy drugs (e.g., bleomycin, topoisomerase II inhibitors such as etoposide and doxorubicin) and radiotherapy commonly used in tumor therapy is to cause lethal double-strand breaks of DNA molecules, and then induce the death of tumor cells. Studies have shown that high expression of DNA-PK is found in tumor tissues treated with chemoradiotherapy, and the increase of DNA-PKcs activity enhances the repair of damaged DNA to a certain extent, prevents tumor cell death, and leads to tolerance to chemoradiotherapy. In addition, the cells that survive in tumor tissues after chemoradiotherapy are often cells with high DNA-PKcs activity that are insensitive to the treatment, which is also the reason for the poor efficacy and poor prognosis. By combining with chemoradiotherapy drugs, DNA-PK inhibitors can inhibit DNA-PKcs activity, thereby greatly reducing tumor DNA repair, inducing cells to enter apoptosis process, and achieving better therapeutic effects.

ATM plays an important role in homologous recombination (HR) repair, and when tumor cells lack ATM due to defects, DNA break repair becomes more dependent on DNA-PKcs-mediated non-homologous end joining (NHEJ) repair for survival. Therefore, DNA-PK inhibitors can also be used as single agents to exert therapeutic effects in tumors with other DNA repair pathway defects.

The present disclosure aims to discover a DNA-PK small molecule inhibitor that can not only be used as a single drug to exert therapeutic effects in tumors with other DNA repair pathway defects. It can also be used in combination with chemoradiotherapy drugs to enhance the sensitivity of tumor tissue to chemoradiotherapy, overcome the problem of drug tolerance, and enhance the inhibitory effect on a variety of solid tumors and hematological tumors.

### CONTENT OF THE PRESENT INVENTION

The present disclosure provides a crystal form A of a compound of formula (I) 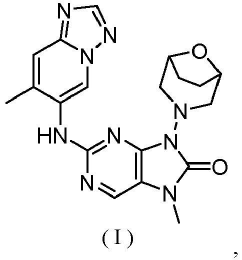 the crystal form A has an X-ray powder diffraction pattern of Cu Kα radiation comprising characteristic diffraction peaks at the following 20 angles: 9.26±0.20°, 19.47±0.20°, 22.69±0.20°.

In some embodiments of the present disclosure, the crystal form A has an X-ray powder diffraction pattern of Cu Kα radiation comprising characteristic diffraction peaks at the following 20 angles: 9.26±0.20°, 11.50±0.20°, 17.03±0.20°, 19.47±0.20°, 22.69±0.20°.

In some embodiments of the present disclosure, the crystal form A has an X-ray powder diffraction pattern of Cu Kα radiation comprising characteristic diffraction peaks at the following 20 angles: 9.26±0.20°, 11.50±0.20°, 13.52±0.20°, 17.03±0.20°, 18.75±0.20°, 19.47±0.20°, 22.69±0.20°, 27.74±0.20°.

In some embodiments of the present disclosure, the crystal form A has an X-ray powder diffraction pattern of Cu Kα radiation comprising characteristic diffraction peaks at the following 20 angles: 5.77±0.20°, 9.26±0.20°, 11.50±0.20°, 13.52±0.20°, 17.03±0.20°, 17.50±0.20°, 18.75±0.20°, 19.47±0.20°, 22.69±0.20°, 23.84±0.20°, 24.42±0.20°, 26.76±0.20°, 27.74±0.20°.

In some embodiments of the present disclosure, the crystal form A has an X-ray powder diffraction pattern of Cu Kα radiation comprising characteristic diffraction peaks at the following 20 angles: 5.77±0.20°, 9.26±0.20°, 11.50±0.20°, 13.52±0.20°, 14.50±0.20°, 15.60±0.20°, 17.03±0.20°, 17.50±0.20°, 18.75±0.20°, 19.47±0.20°, 22.69±0.20°, 23.84±0.20°, 24.42±0.20°, 26.76±0.20°, 27.74±0.20°, 30.40±0.20°.

In some embodiments of the present disclosure, the crystal form A has an X-ray powder diffraction pattern of Cu Kα radiation comprising characteristic diffraction peaks at the following 20 angles: 9.26±0.20°, 19.47±0.20°, 22.69±0.20°, and/ or 5.77±0.20°, and/ or 11.50±0.20°, and/ or 13.52±0.20°, and/ or 14.50±0.20°, and/ or 15.16±0.20°, and/ or 15.60±0.20°, and/ or 17.03±0.20°, and/ or 17.28±0.20°, and/ or 17.50±0.20°, and/ or 18.47±0.20°, and/ or 18.75±0.20°, and/ or 19.27±0.20°, and/ or 23.84±0.20°, and/ or 24.42±0.20°, and/ or 26.76±0.20°, and/ or 27.74±0.20°, and/ or 28.43±0.20°, and/ or 29.77±0.20°, and/ or 30.40±0.20°, and/ or 32.08±0.20°.

In some embodiments of the present disclosure, the crystal form A has an X-ray powder diffraction pattern of Cu Kα radiation comprising characteristic diffraction peaks at the following 20 angles: 5.77°, 9.26°, 11.50°, 13.52°, 14.50°, 15.16°, 15.60°, 17.03°, 17.28°, 17.50°, 18.47°, 18.75°, 19.27°, 19.47°, 22.69°, 23.84°, 24.42°, 26.76°, 27.74°, 28.43°, 29.77°, 30.40°, 32.08°.

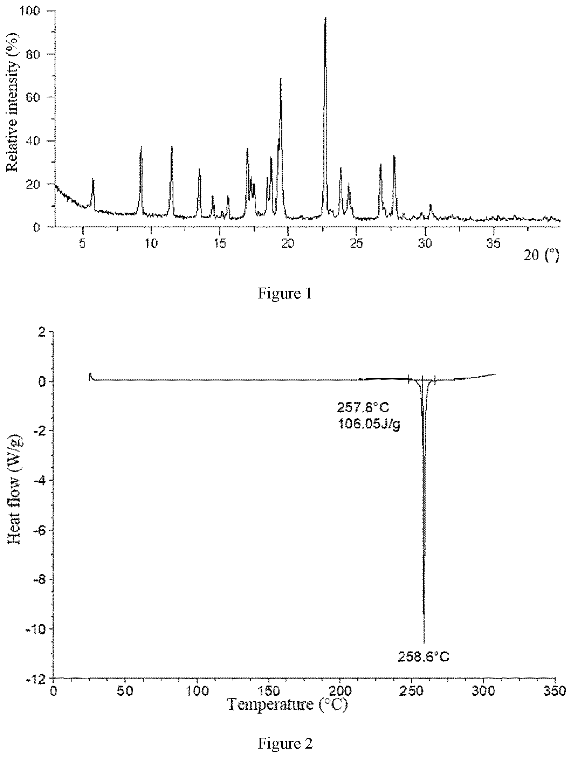

In some embodiments of the present disclosure, the crystal form A has an XRPD pattern as shown in Figure 1.

In some embodiments of the present disclosure, the crystal form A has an XRPD pattern basically as shown in Figure 1.

In some embodiments of the present disclosure, the X-ray powder diffraction pattern of Cu Kα radiation of the crystal form A has peak positions and relative intensities of diffraction peaks as shown in the table below:

**Table 1 XRPD diffraction data of the crystal form A of the compound of formula (I)**

| **No.** | **Diffraction angle 2θ** | **Peak height** | **Relative intensity (%)** | **No.** | **Diffraction angle 2θ** | **Peak height** | **Relative intensity (%)** |
|---|---|---|---|---|---|---|---|
| 1 | 5.77 | 586.27 | 15.42 | 13 | 19.27 | 1428.42 | 37.56 |
| 2 | 9.26 | 1316.63 | 34.62 | 14 | 19.47 | 2536.63 | 66.71 |
| 3 | 11.50 | 1326.39 | 34.88 | 15 | 22.69 | 3802.68 | 100.00 |
| 4 | 13.52 | 947.57 | 24.92 | 16 | 23.84 | 967.59 | 25.44 |
| 5 | 14.50 | 409.18 | 10.76 | 17 | 24.42 | 686.03 | 18.04 |
| 6 | 15.16 | 129.18 | 3.40 | 18 | 26.76 | 1068.12 | 28.09 |
| 7 | 15.60 | 427.66 | 11.25 | 19 | 27.74 | 1226.40 | 32.25 |
| 8 | 17.03 | 1342.38 | 35.30 | 20 | 28.43 | 101.75 | 2.68 |
| 9 | 17.28 | 788.64 | 20.74 | 21 | 29.77 | 111.22 | 2.92 |
| 10 | 17.50 | 656.55 | 17.27 | 22 | 30.40 | 298.34 | 7.85 |
| 11 | 18.47 | 795.01 | 20.91 | 23 | 32.08 | 48.53 | 1.28 |
| 12 | 18.75 | 1206.93 | 31.74 | - | - | | - |

In some embodiments of the present disclosure, the crystal form A has a differential scanning calorimetry (DSC) curve showing an endothermic peak with an onset at 257.8°C ± 3°C.

In some embodiments of the present disclosure, the crystal form A has a differential scanning calorimetry (DSC) curve showing an endothermic peak with an onset at 257.8°C ± 5°C.

In some embodiments of the present disclosure, the crystal form A has a DSC pattern as shown in Figure 2.

In some embodiments of the present disclosure, the crystal form A has a DSC pattern basically as shown in Figure 2.

In some embodiments of the present disclosure, the crystal form A has a thermogravimetric analysis (TGA) curve with a weight loss of 1.39% at 230°C±3°C.

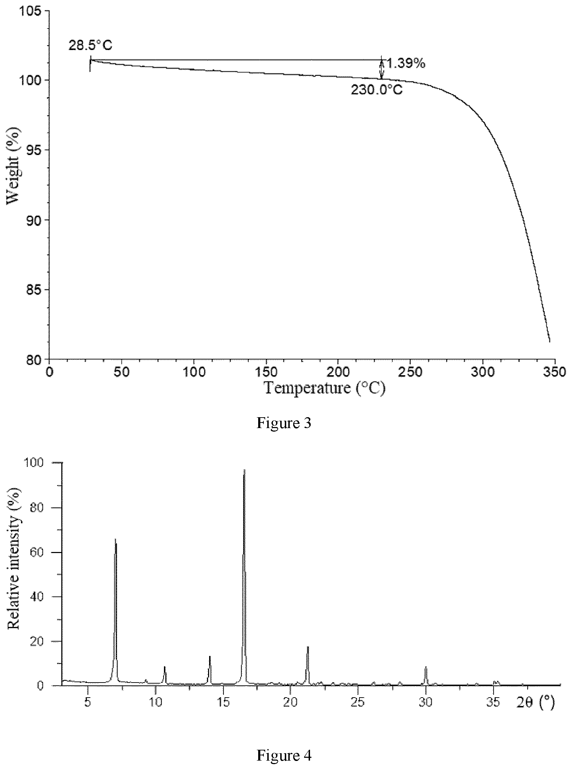

In some embodiments of the present disclosure, the crystal form A has a TGA pattern as shown in Figure 3.

In some embodiments of the present disclosure, the crystal form A has a TGA pattern basically as shown in Figure 3.

The present disclosure also provides a mesylate and a *p*-toluenesulfonate of a compound of formula (I),

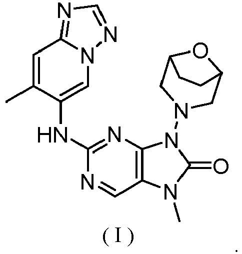

In some embodiments of the present disclosure, the methanesulfonate of the compound of formula (I) has a structure represented by formula (II), and the *p*-toluenesulfonate of the compound of formula (I) has a structure represented by formula (III), 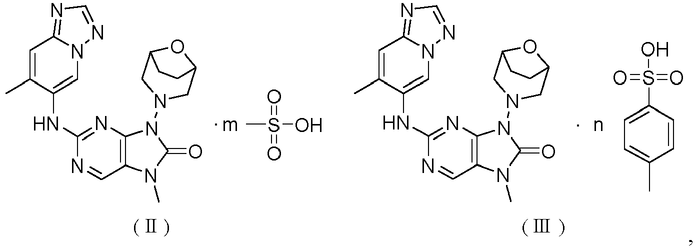 wherein m and n are independently selected from 0.6 to 2.5, respectively.

In some embodiments of the present disclosure, the methanesulfonate of the compound of formula (I) has a structure represented by formula (II), and the *p*-toluenesulfonate of the compound of formula (I) has a structure represented by formula (III), 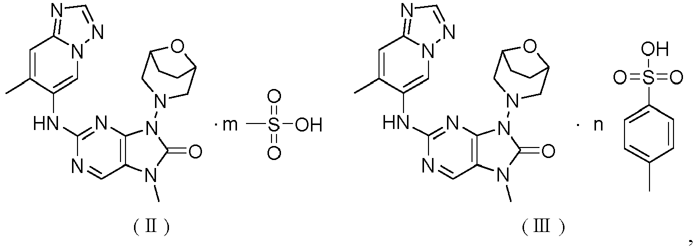 wherein
m is selected from 0.8, 0.9, 1.0, 1.1, 1.2, 1.5, 1.8, 1.9, 2.0, 2.1 and 2.2;
n is selected from 0.8, 0.9, 1.0, 1.1, 1.2, 1.5, 1.8, 1.9, 2.0, 2.1 and 2.2.

In some embodiments of the present disclosure, m is selected from 1.0, 2.0 and 2.2.

In some embodiments of the present disclosure, n is selected from 1.0, 2.0 and 2.1.

In some embodiments of the present disclosure, the compound of formula (II) is selected from a compound of formula (II-1), and the compound of formula (III) is selected from a compound of formula (III-1),

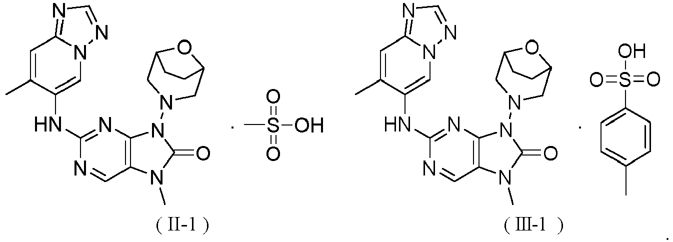

The present disclosure also provides a crystal form B of a compound of formula (II-1), 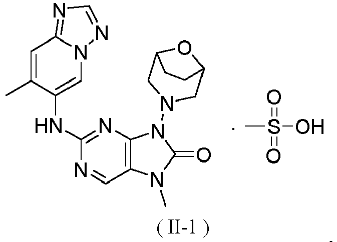 the crystal form B has an X-ray powder diffraction pattern of Cu Kα radiation comprising characteristic diffraction peaks at the following 20 angles: 7.02±0.20°, 16.56±0.20°, 21.25±0.20°.

In some embodiments of the present disclosure, the crystal form B has an X-ray powder diffraction pattern of Cu Kα radiation comprising characteristic diffraction peaks at the following 20 angles: 7.02±0.20°, 10.66±0.20°, 13.98±0.20°, 16.56±0.20°, 21.25±0.20°.

In some embodiments of the present disclosure, the crystal form B has an X-ray powder diffraction pattern of Cu Kα radiation comprising characteristic diffraction peaks at the following 20 angles: 7.02±0.20°, 10.66±0.20°, 13.98±0.20°, 16.56±0.20°, 21.25±0.20°, 29.98±0.20°.

In some embodiments of the present disclosure, the crystal form B has an X-ray powder diffraction pattern of Cu Kα radiation comprising characteristic diffraction peaks at the following 20 angles: 7.02±0.20°, 9.29±0.20°, 10.66±0.20°, 13.98±0.20°, 16.56±0.20°, 21.25±0.20°, 29.98±0.20°.

In some embodiments of the present disclosure, the crystal form B has an X-ray powder diffraction pattern of Cu Kα radiation comprising characteristic diffraction peaks at the following 20 angles: 7.02±0.20°, 9.29±0.20°, 10.66±0.20°, 13.98±0.20°, 16.56±0.20°, 20.51±0.20°, 21.25±0.20°, 26.14±0.20°, 28.08±0.20°, 29.98±0.20°.

In some embodiments of the present disclosure, the crystal form B has an X-ray powder diffraction pattern of Cu Kα radiation comprising characteristic diffraction peaks at the following 20 angles: 7.02±0.20°, 9.29±0.20°, 10.66±0.20°, 13.98±0.20°, 16.56±0.20°, 20.51±0.20°, 21.25±0.20°, 26.14±0.20°, 28.08±0.20°, 29.98±0.20°, 35.07±0.20°.

In some embodiments of the present disclosure, the crystal form B has an X-ray powder diffraction pattern of Cu Kα radiation comprising characteristic diffraction peaks at the following 20 angles: 7.02±0.20°, 9.29±0.20°, 10.66±0.20°, 13.98±0.20°, 16.56±0.20°, 18.54±0.20°, 19.11±0.20°, 20.51±0.20°, 21.25±0.20°, 22.25±0.20°, 23.14±0.20°, 23.77±0.20°, 26.14±0.20°, 28.08±0.20°, 29.98±0.20°.

In some embodiments of the present disclosure, the crystal form B has an X-ray powder diffraction pattern of Cu Kα radiation comprising characteristic diffraction peaks at the following 20 angles: 7.02±0.20°, 9.29±0.20°, 10.66±0.20°, 13.98±0.20°, 16.56±0.20°, 18.54±0.20°, 19.11±0.20°, 20.51±0.20°, 21.25±0.20°, 22.25±0.20°, 23.14±0.20°, 23.77±0.20°, 26.14±0.20°, 28.08±0.20°, 29.98±0.20°, 35.07±0.20°.

In some embodiments of the present disclosure, the crystal form B has an X-ray powder diffraction pattern of Cu Kα radiation comprising characteristic diffraction peaks at the following 20 angles: 7.02±0.20°, 16.56±0.20°, 21.25±0.20°, and/or 13.98±0.20°, and/or 10.66±0.20°, and/or 29.98±0.20°, and/or 9.29±0.20°, and/or 26.14±0.20°, and/or 20.51±0.20°, and/or 30.57±0.20°, and/or 35.33±0.20°, and/or 28.08±0.20°, and/or 22.25±0.20°, and/or 23.14±0.20°.

In some embodiments of the present disclosure, the crystal form B has an X-ray powder diffraction pattern of Cu Kα radiation comprising characteristic diffraction peaks at the following 20 angles: 7.02°, 9.29°, 10.66°, 13.98°, 16.56°, 18.54°, 19.11°, 20.51°, 21.25°, 21.98°, 22.25°, 23.14°, 23.77°, 26.14°, 27.27°, 28.08°, 29.98°.

In some embodiments of the present disclosure, the crystal form B has an X-ray powder diffraction pattern of Cu Kα radiation comprising characteristic diffraction peaks at the following 20 angles: 7.02°, 9.29°, 10.66°, 13.98°, 16.56°, 18.54°, 19.11°, 20.51°, 21.25°, 21.98°, 22.25°, 23.14°, 23.77°, 26.14°, 27.27°, 28.08°, 29.98°, 30.67°, 33.75°, 35.07°, 35.33°, 37.17°.

In some embodiments of the present disclosure, the crystal form B has an XRPD pattern as shown in Figure 4.

In some embodiments of the present disclosure, the crystal form B has an XRPD pattern basically as shown in Figure 4.

In some embodiments of the present disclosure, the X-ray powder diffraction pattern of Cu Kα radiation of the crystal form B has peak positions and relative intensities of diffraction peaks as shown in the table below:

**Table 2 XRPD diffraction data of the crystal form B of the compound of formula (II-1)**

| **No.** | **Diffraction angle 2θ** | **Peak height** | **Relative intensity (%)** | **No.** | **Diffraction angle 2θ** | **Peak height** | **Relative intensity (%)** |
|---|---|---|---|---|---|---|---|
| 1 | 7.02 | 17003.86 | 65.93 | 12 | 23.14 | 262.20 | 1.02 |
| 2 | 9.29 | 417.16 | 1.62 | 13 | 23.77 | 222.80 | 0.86 |
| 3 | 10.66 | 2082.57 | 8.07 | 14 | 26.14 | 333.95 | 1.29 |
| 4 | 13.98 | 3377.68 | 13.10 | 15 | 27.27 | 120.81 | 0.47 |
| 5 | 16.56 | 25791.82 | 100.00 | 16 | 28.08 | 327.37 | 1.27 |
| 6 | 18.54 | 226.48 | 0.88 | 17 | 29.98 | 2120.07 | 8.22 |
| 7 | 19.11 | 199.53 | 0.77 | 18 | 30.67 | 163.54 | 0.63 |
| 8 | 20.51 | 303.83 | 1.18 | 19 | 33.75 | 134.07 | 0.52 |
| 9 | 21.25 | 4534.37 | 17.58 | 20 | 35.07 | 437.00 | 1.69 |
| 10 | 21.98 | 202.05 | 0.78 | 21 | 35.33 | 432.24 | 1.68 |
| 11 | 22.25 | 273.41 | 1.06 | 22 | 37.17 | 87.96 | 0.34 |

In some embodiments of the present disclosure, the crystal form B has a differential scanning calorimetry (DSC) curve showing an endothermic peak with an onset at 286.6°C ± 3°C.

In some embodiments of the present disclosure, the crystal form B has a differential scanning calorimetry (DSC) curve showing an endothermic peak with an onset at 286.6°C ± 5°C.

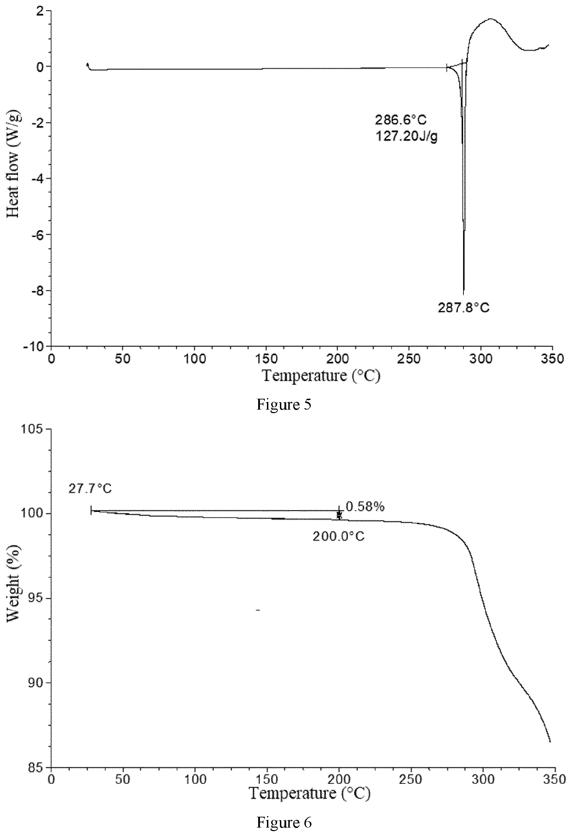

In some embodiments of the present disclosure, the crystal form B has a DSC pattern as shown in Figure 5.

In some embodiments of the present disclosure, the crystal form B has a DSC pattern basically as shown in Figure 5.

In some embodiments of the present disclosure, the crystal form B has a thermogravimetric analysis (TGA) curve with a weight loss of 0.58% at 200°C±3°C.

In some embodiments of the present disclosure, the crystal form B has a TGA pattern as shown in Figure 6.

In some embodiments of the present disclosure, the crystal form B has a TGA pattern basically as shown in Figure 6.

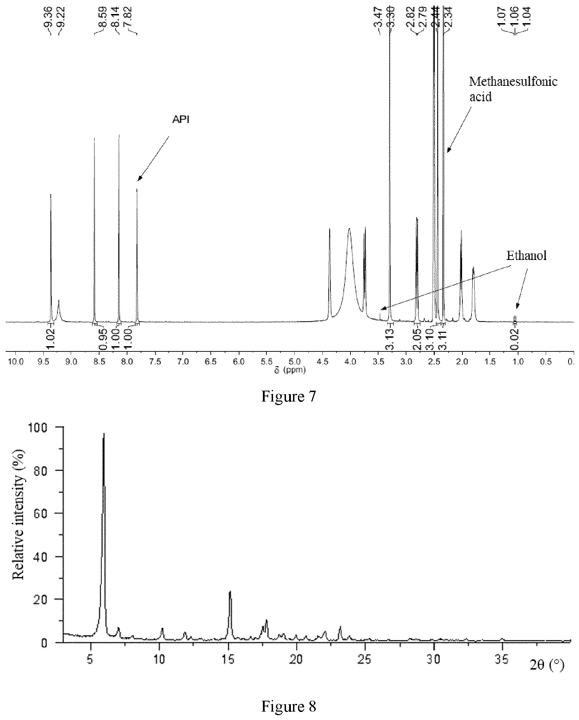

In some embodiments of the present disclosure, the crystal form B has a ¹H NMR pattern as shown in Figure 7.

In some embodiments of the present disclosure, the crystal form B has a ¹H NMR pattern basically as shown in Figure 7.

The present disclosure also provides a preparation method for the crystal form B, which comprises the following steps:
(a) compound Z is added to solvent X;
(b) methanesulfonic acid is added dropwise at 25°C to 80°C, and the mixture is stirred at 25°C to 80°C for 3 to 16 hours after addition;
(c) solvent Y is added dropwise, and the mixture is stirred at 25°C to 80°C for 1 to 3 hours after addition;
(d) the mixture is cooled down to 15°C to 30°C;
(e) the mixture is filtered;
(g) the residue is dried under vacuum at 25°C to 60°C for 1 to 24 hours;
wherein,
the compound Z is selected from the compound of formula (I), the crystal form A of the compound of formula (I), and the crystal form K of the compound of formula (VIII-1);
the solvent X is selected from dimethyl sulfoxide, methanol, ethanol, acetonitrile, acetone, tetrahydrofuran and dichloromethane;
the solvent Y is absent, or the solvent Y is selected from methyl *tert*-butyl ether, ethyl acetate and *n*-heptane;
the methanesulfonic acid and the crystal form A of the compound of formula (I) has a molar ratio of 1.0:1 to 1.2:1.

The present disclosure also provides a crystal form C of a compound of formula (III-1), 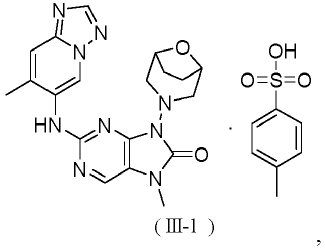 the crystal form C has an X-ray powder diffraction pattern of Cu Kα radiation comprising characteristic diffraction peaks at the following 20 angles: 5.96±0.20°, 15.16±0.20°, 17.83±0.20°.

In some embodiments of the present disclosure, the crystal form C has an X-ray powder diffraction pattern of Cu Kα radiation comprising characteristic diffraction peaks at the following 20 angles: 5.96±0.20°, 10.23±0.20°, 15.16±0.20°, 17.83±0.20°, 23.19±0.20°.

In some embodiments of the present disclosure, the crystal form C has an X-ray powder diffraction pattern of Cu Kα radiation comprising characteristic diffraction peaks at the following 20 angles: 5.96±0.20°, 7.05±0.20°, 10.23±0.20°, 15.16±0.20°, 17.83±0.20°, 22.11±0.20°, 23.19±0.20°.

In some embodiments of the present disclosure, the crystal form C has an X-ray powder diffraction pattern of Cu Kα radiation comprising characteristic diffraction peaks at the following 20 angles: 5.96±0.20°, 7.05±0.20°, 10.23±0.20°, 11.89±0.20°, 15.16±0.20°, 17.83±0.20°, 19.09±0.20°, 19.96±0.20°, 22.11±0.20°, 23.19±0.20°.

In some embodiments of the present disclosure, the crystal form C has an X-ray powder diffraction pattern of Cu Kα radiation comprising characteristic diffraction peaks at the following 20 angles: 5.96±0.20°, 7.05±0.20°, 10.23±0.20°, 11.89±0.20°, 15.16±0.20°, 16.67±0.20°, 17.54±0.20°, 17.83±0.20°, 18.75±0.20°, 19.09±0.20°, 19.96±0.20°, 20.67±0.20°, 21.6±0.20°, 22.11±0.20°, 23.19±0.20°, 23.83±0.20°.

In some embodiments of the present disclosure, the crystal form C has an X-ray powder diffraction pattern of Cu Kα radiation comprising characteristic diffraction peaks at the following 20 angles: 5.96±0.20°, 15.16±0.20°, 17.83±0.20°, and/or 7.05±0.20°, and/or 8.08±0.20°, and/or 10.23±0.20°, and/or 11.89±0.20°, and/or 12.30±0.20°, and/or 12.99±0.20°, and/or 14.01±0.20°, and/or 16.67±0.20°, and/or 17.54±0.20°, and/or 18.75±0.20°, and/or 19.09±0.20°, and/or 19.47±0.20°, and/or 19.96±0.20°, and/or 20.67±0.20°, and/or 21.6±0.20°, and/or 22.11±0.20°, and/or 23.19±0.20°, and/or 23.83±0.20°, and/or 25.18±0.20°, and/or 26.69±0.20°, and/or 28.32±0.20°, and/or 29.90±0.20°, and/or 30.48±0.20°, and/or 32.39±0.20°, and/or 35.02±0.20°.

In some embodiments of the present disclosure, the crystal form C has an X-ray powder diffraction pattern of Cu Kα radiation comprising characteristic diffraction peaks at the following 20 angles: 5.96°, 7.05°, 8.08°, 10.23°, 11.89°, 12.30°, 12.99°, 14.01°, 15.16°, 16.67°, 17.54°, 17.83°, 18.75°, 19.09°, 19.47°, 19.96°, 20.67°, 21.6°, 22.11°, 23.19°, 23.83°, 25.18°, 26.69°, 28.32°, 29.90°, 30.48°, 32.39°, 35.02°.

In some embodiments of the present disclosure, the crystal form C has an XRPD pattern as shown in Figure 8.

In some embodiments of the present disclosure, the crystal form C has an XRPD pattern basically as shown in Figure 8.

In some embodiments of the present disclosure, the X-ray powder diffraction pattern of Cu Kα radiation of the crystal form C has peak positions and relative intensities of diffraction peaks as shown in the table below:

**Table 3 XRPD diffraction data of the crystal form C of the compound of formula (III-1)**

| **No.** | **Diffraction angle 2θ** | **Peak height** | **Relative intensity (%)** | **No.** | **Diffraction angle 2θ** | **Peak height** | **Relative intensity (%)** |
|---|---|---|---|---|---|---|---|
| 1 | 5.96 | 16844.73 | 100.00 | 15 | 19.47 | 149.60 | 0.89 |
| 2 | 7.05 | 875.78 | 5.20 | 16 | 19.96 | 468.03 | 2.78 |
| 3 | 8.08 | 196.16 | 1.16 | 17 | 20.67 | 387.01 | 2.30 |
| 4 | 10.23 | 980.16 | 5.82 | 18 | 21.60 | 332.62 | 1.97 |
| 5 | 11.89 | 635.46 | 3.77 | 19 | 22.11 | 752.03 | 4.46 |
| 6 | 12.30 | 279.05 | 1.66 | 20 | 23.19 | 1132.61 | 6.72 |
| 7 | 12.99 | 153.38 | 0.91 | 21 | 23.83 | 356.85 | 2.12 |
| 8 | 14.01 | 93.76 | 0.56 | 22 | 25.18 | 113.71 | 0.68 |
| 9 | 15.16 | 4151.59 | 24.65 | 23 | 26.69 | 78.69 | 0.47 |
| 10 | 16.67 | 326.15 | 1.94 | 24 | 28.32 | 163.25 | 0.97 |
| 11 | 17.54 | 1155.51 | 6.86 | 25 | 29.90 | 140.55 | 0.83 |
| 12 | 17.83 | 1701.96 | 10.10 | 26 | 30.48 | 113.78 | 0.68 |
| 13 | 18.75 | 465.76 | 2.76 | 27 | 32.39 | 136.59 | 0.81 |
| 14 | 19.09 | 549.77 | 3.26 | 28 | 35.02 | 151.07 | 0.90 |

In some embodiments of the present disclosure, the crystal form C has a differential scanning calorimetry (DSC) curve showing an endothermic peak with an onset at 255.4 ± 3°C.

In some embodiments of the present disclosure, the crystal form C has a differential scanning calorimetry (DSC) curve showing an endothermic peak with an onset at 255.4 ± 5°C.

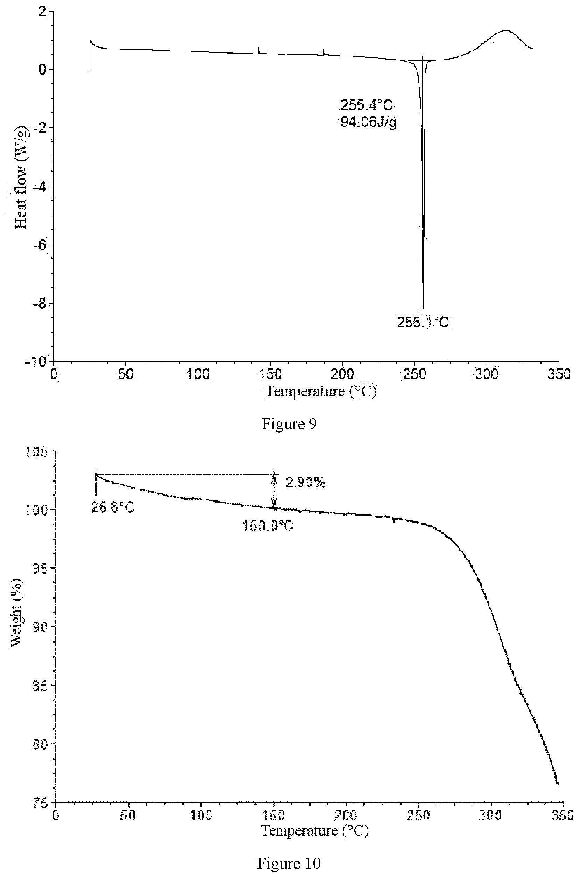

In some embodiments of the present disclosure, the crystal form C has a DSC pattern as shown in Figure 9.

In some embodiments of the present disclosure, the crystal form C has a DSC pattern basically as shown in Figure 9.

In some embodiments of the present disclosure, the crystal form C has a thermogravimetric analysis (TGA) curve with a weight loss of 2.90% at 150°C±3°C.

In some embodiments of the present disclosure, the crystal form C has a TGA pattern as shown in Figure 10.

In some embodiments of the present disclosure, the crystal form C has a TGA pattern basically as shown in Figure 10.

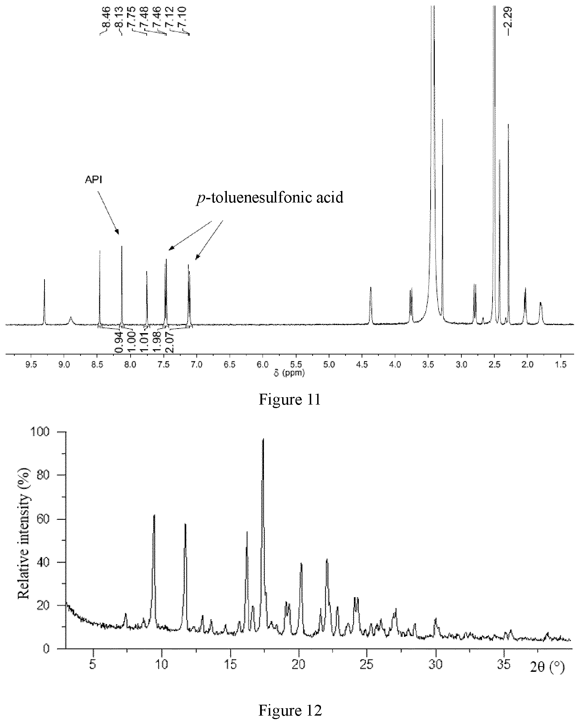

In some embodiments of the present disclosure, the crystal form C has a ¹H NMR pattern basically as shown in Figure 11.

In some embodiments of the present disclosure, the crystal form C has a ¹H NMR pattern basically as shown in Figure 11.

The present disclosure also provides a preparation method for the crystal form C, which comprises the following steps:
(a) the crystal form K (1 *eq*) of the compound of formula (VIII-1) and *p-*toluenesulfonic acid (1 *eq*) are added to into tetrahydrofuran;
(b) the suspension is stirred at room temperature for 2 days;
(c) the suspension is centrifuged, and the solid is collected and dried under vacuum at room temperature for 3 hours.

In some embodiments of the present disclosure, the compound of formula (II) or formula (III) is selected from,

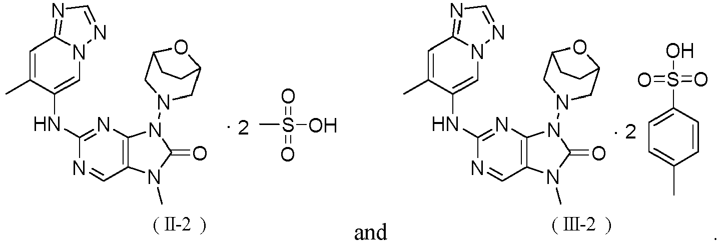

The present disclosure also provides a crystal form D of a compound of formula (II-2), 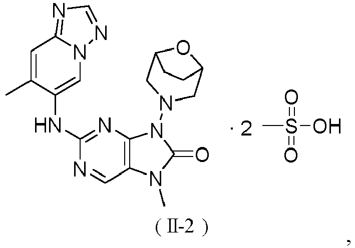 the crystal form D has an X-ray powder diffraction pattern of Cu Kα radiation comprising characteristic diffraction peaks at the following 20 angles: 9.45±0.20°, 11.75±0.20°, 17.41±0.20°.

In some embodiments of the present disclosure, the crystal form D has an X-ray powder diffraction pattern of Cu Kα radiation comprising characteristic diffraction peaks at the following 20 angles: 9.45±0.20°, 11.75±0.20°, 16.23±0.20°, 17.41±0.20°, 20.21±0.20°.

In some embodiments of the present disclosure, the crystal form D has an X-ray powder diffraction pattern of Cu Kα radiation comprising characteristic diffraction peaks at the following 20 angles: 9.45±0.20°, 11.75±0.20°, 16.23±0.20°, 17.41±0.20°, 19.10±0.20°, 20.21±0.20°, 22.06±0.20°, 24.32±0.20°.

In some embodiments of the present disclosure, the crystal form D has an X-ray powder diffraction pattern of Cu Kα radiation comprising characteristic diffraction peaks at the following 20 angles: 7.39±0.20°, 9.45±0.20°, 11.75±0.20°, 12.99±0.20°, 16.23±0.20°, 17.41±0.20°, 19.10±0.20°, 20.21±0.20°, 22.06±0.20°, 24.32±0.20°, 22.82±0.20°.

In some embodiments of the present disclosure, the crystal form D has an X-ray powder diffraction pattern of Cu Kα radiation comprising characteristic diffraction peaks at the following 20 angles: 7.39±0.20°, 9.45±0.20°, 11.75±0.20°, 12.99±0.20°, 16.23±0.20°, 16.70±0.20°, 17.41±0.20°, 17.64±0.20°, 19.10±0.20°, 20.21±0.20°, 21.62±0.20°, 22.06±0.20°, 22.82±0.20°, 24.32±0.20°, 27.09±0.20°, 29.99±0.20°.

In some embodiments of the present disclosure, the crystal form D has an X-ray powder diffraction pattern of Cu Kα radiation comprising characteristic diffraction peaks at the following 20 angles: 9.45±0.20°, 11.75±0.20°, 17.41±0.20°, and/or 7.39±0.20°, and/or 8.69±0.20°, and/or 12.99±0.20°, and/or 13.62±0.20°, and/or 14.65±0.20°, and/or 15.68±0.20°, and/or 16.23±0.20°, and/or 16.7±0.20°, and/or 17.64±0.20°, and/or 18.02±0.20°, and/or 19.1±0.20°, and/or 19.33±0.20°, and/or 20.21±0.20°, and/or 21.62±0.20°, and/or 22.06±0.20°, and/or 22.82±0.20°, and/or 23.57±0.20°, and/or 24.08±0.20°, and/or 24.32±0.20°, and/or 25.29±0.20°, and/or 26.02±0.20°, and/or 27.09±0.20°, and/or 28.02±0.20°, and/or 28.51±0.20°, and/or 29.99±0.20°, and/or 32.18±0.20°, and/or 35.13±0.20°, and/or 35.43±0.20°, and/or 38.16±0.20°.

In some embodiments of the present disclosure, the crystal form D has an X-ray powder diffraction pattern of Cu Kα radiation comprising characteristic diffraction peaks at the following 20 angles: 7.39°, 8.69°, 9.45°, 11.75°, 12.99°, 13.62°, 14.65°, 15.68°, 16.23°, 16.7°, 17.41°, 17.64°, 18.02°, 19.1°, 19.33°, 20.21°, 21.62°, 22.06°, 22.82°, 23.57°, 24.08°, 24.32°, 25.29°, 26.02°, 27.09°, 28.02°, 28.51°, 29.99°, 32.18°, 35.13°, 35.43°, 38.16°.

In some embodiments of the present disclosure, the crystal form D has an XRPD pattern as shown in Figure 12.

In some embodiments of the present disclosure, the crystal form D has an XRPD pattern basically as shown in Figure 12.

In some embodiments of the present disclosure, the X-ray powder diffraction pattern of Cu Kα radiation of the crystal form D has peak positions and relative intensities of diffraction peaks as shown in the table below:

**Table 4 XRPD diffraction data of the crystal form D of the compound of formula (II-2)**

| **No.** | **Diffraction angle 2θ** | **Peak height** | **Relative intensity (%)** | **No.** | **Diffraction angle 2θ** | **Peak height** | **Relative intensity (%)** |
|---|---|---|---|---|---|---|---|
| 1 | 7.39 | 238.42 | 7.43 | 17 | 21.62 | 434.32 | 13.53 |
| 2 | 8.69 | 146.37 | 4.56 | 18 | 22.06 | 1281.55 | 39.93 |
| 3 | 9.45 | 1873.51 | 58.38 | 19 | 22.82 | 483.21 | 15.06 |
| 4 | 11.75 | 1767.36 | 55.07 | 20 | 23.57 | 212.23 | 6.61 |
| 5 | 12.99 | 262.23 | 8.17 | 21 | 24.08 | 627.59 | 19.56 |
| 6 | 13.62 | 190.14 | 5.92 | 22 | 24.32 | 651.33 | 20.30 |
| 7 | 14.65 | 105.20 | 3.28 | 23 | 25.29 | 223.58 | 6.97 |
| 8 | 15.68 | 195.17 | 6.08 | 24 | 26.02 | 293.31 | 9.14 |
| 9 | 16.23 | 1623.03 | 50.57 | 25 | 27.09 | 428.97 | 13.37 |
| 10 | 16.70 | 449.70 | 14.01 | 26 | 28.02 | 134.01 | 4.18 |
| 11 | 17.41 | 3209.30 | 100.00 | 27 | 28.51 | 206.68 | 6.44 |
| 12 | 17.64 | 658.23 | 20.51 | 28 | 29.99 | 303.95 | 9.47 |
| 13 | 18.02 | 209.50 | 6.53 | 29 | 32.18 | 100.28 | 3.12 |
| 14 | 19.10 | 561.28 | 17.49 | 30 | 35.13 | 108.14 | 3.37 |
| 15 | 19.33 | 500.40 | 15.59 | 31 | 35.43 | 116.31 | 3.62 |
| 16 | 20.21 | 1177.56 | 36.69 | - | 38.16 | 99.52 | 3.10 |

In some embodiments of the present disclosure, the crystal form D has a differential scanning calorimetry (DSC) curve showing endothermic peaks with onsets at 43.1°C ± 3°C and 227.4°C ± 3°C.

In some embodiments of the present disclosure, the crystal form D has a differential scanning calorimetry (DSC) curve showing an endothermic peak with an onset at 227.4°C ± 5°C.

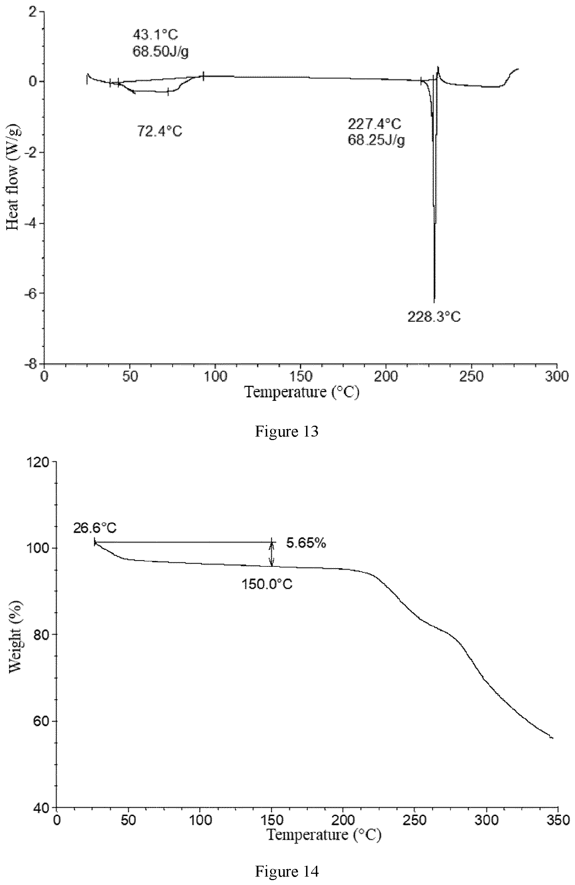

In some embodiments of the present disclosure, the crystal form D has a DSC pattern as shown in Figure 13.

In some embodiments of the present disclosure, the crystal form D has a DSC pattern basically as shown in Figure 13.

In some embodiments of the present disclosure, the crystal form D has a thermogravimetric analysis (TGA) curve with a weight loss of 5.65% at 150°C±3°C.

In some embodiments of the present disclosure, the crystal form D has a TGA pattern as shown in Figure 14.

In some embodiments of the present disclosure, the crystal form D has a TGA pattern basically as shown in Figure 14.

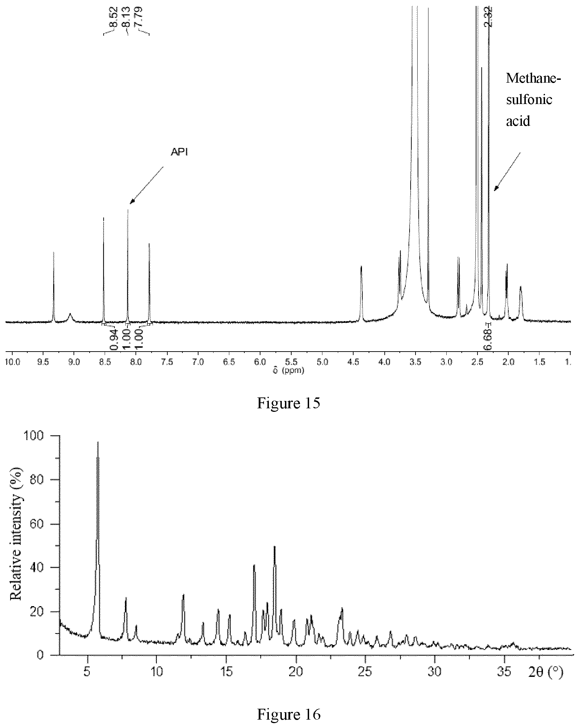

In some embodiments of the present disclosure, the crystal form D has a ¹H NMR pattern as shown in Figure 15.

In some embodiments of the present disclosure, the crystal form D has a ¹H NMR pattern basically as shown in Figure 15.

The present disclosure also provides a crystal form E of a compound of formula (III-2), 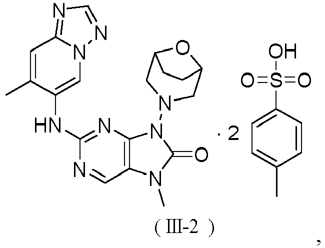 the crystal form E has an X-ray powder diffraction pattern of Cu Kα radiation comprising characteristic diffraction peaks at the following 20 angles: 5.78±0.20°, 17.02±0.20°, 18.49±0.20°.

In some embodiments of the present disclosure, the crystal form E has an X-ray powder diffraction pattern of Cu Kα radiation comprising characteristic diffraction peaks at the following 20 angles: 5.78±0.20°, 7.77±0.20°, 11.90±0.20°, 17.02±0.20°, 17.97±0.20°, 18.49±0.20°, 18.95±0.20°, 23.33±0.20°.

In some embodiments of the present disclosure, the crystal form E has an X-ray powder diffraction pattern of Cu Kα radiation comprising characteristic diffraction peaks at the following 20 angles: 5.78±0.20°, 7.77±0.20°, 11.90±0.20°, 14.43±0.20°, 15.24±0.20°, 17.02±0.20°, 17.97±0.20°, 18.49±0.20°, 18.95±0.20°, 21.06±0.20°, 23.33±0.20°.

In some embodiments of the present disclosure, the crystal form E has an X-ray powder diffraction pattern of Cu Kα radiation comprising characteristic diffraction peaks at the following 20 angles: 5.78±0.20°, 17.02±0.20°, 18.49±0.20°, and/or 7.77±0.20°, and/or 8.50±0.20°, and/or 11.90±0.20°, and/or 13.34±0.20°, and/or 14.43±0.20°, and/or 15.24±0.20°, and/or 16.38±0.20°, and/or 17.67±0.20°, and/or 17.97±0.20°, and/or 18.95±0.20°, and/or 19.87±0.20°, and/or 20.78±0.20°, and/or 21.06±0.20°, and/or 21.66±0.20°, and/or 21.90±0.20°, and/or 23.04±0.20°, and/or 23.33±0.20°, and/or 23.89±0.20°, and/or 24.45±0.20°, and/or 24.84±0.20°, and/or 25.85±0.20°, and/or 26.82±0.20°, and/or 27.97±0.20°, and/or 28.65±0.20°, and/or 29.92±0.20°, and/or 30.25±0.20°, and/or 31.22±0.20°, and/or 33.01±0.20°, and/or 33.85±0.20°, and/or 35.61±0.20°.

In some embodiments of the present disclosure, the crystal form E has an X-ray powder diffraction pattern of Cu Kα radiation comprising characteristic diffraction peaks at the following 20 angles: 5.78°, 7.77°, 8.50°, 11.90°, 13.34°, 14.43°, 15.24°, 16.38°, 17.02°, 17.67°, 17.97°, 18.49°, 18.95°, 19.87°, 20.78°, 21.06°, 21.66°, 21.90°, 23.04°, 23.33°, 23.89°, 24.45°, 24.84°, 25.85°, 26.82°, 27.97°, 28.65°, 29.92°, 30.25°, 31.22°, 33.01°, 33.85°, 35.61°.

In some embodiments of the present disclosure, the crystal form E has an XRPD pattern as shown in Figure 16.

In some embodiments of the present disclosure, the crystal form E has an XRPD pattern basically as shown in Figure 16.

In some embodiments of the present disclosure, the X-ray powder diffraction pattern of Cu Kα radiation of the crystal form E has peak positions and relative intensities of diffraction peaks as shown in the table below:

**Table 5 XRPD diffraction data of the crystal form E of the compound of formula (III-2)**

| **No.** | **Diffraction angle 2θ** | **Peak height** | **Relative intensity (%)** | **No.** | **Diffraction angle 2θ** | **Peak height** | **Relative intensity (%)** |
|---|---|---|---|---|---|---|---|
| 1 | 5.78 | 4072.98 | 100.00 | 18 | 21.90 | 160.02 | 3.93 |
| 2 | 7.77 | 877.37 | 21.54 | 19 | 23.04 | 479.05 | 11.76 |
| 3 | 8.50 | 323.93 | 7.95 | 20 | 23.33 | 792.90 | 19.47 |
| 4 | 11.90 | 1007.68 | 24.74 | 21 | 23.89 | 307.99 | 7.56 |
| 5 | 13.34 | 443.31 | 10.88 | 22 | 24.45 | 355.08 | 8.72 |
| 6 | 14.43 | 718.73 | 17.65 | 23 | 24.84 | 203.16 | 4.99 |
| 7 | 15.24 | 617.91 | 15.17 | 24 | 25.85 | 203.46 | 5.00 |
| 8 | 16.38 | 252.49 | 6.20 | 25 | 26.82 | 348.10 | 8.55 |
| 9 | 17.02 | 1712.80 | 42.05 | 26 | 27.97 | 258.75 | 6.35 |
| 10 | 17.67 | 720.89 | 17.70 | 27 | 28.65 | 207.34 | 5.09 |
| 11 | 17.97 | 883.45 | 21.69 | 28 | 29.92 | 113.17 | 2.78 |
| 12 | 18.49 | 2073.96 | 50.92 | 29 | 30.25 | 101.06 | 2.48 |
| 13 | 18.95 | 759.21 | 18.64 | 30 | 31.22 | 87.48 | 2.15 |
| 14 | 19.87 | 519.82 | 12.76 | 31 | 33.01 | 38.44 | 0.94 |
| 15 | 20.78 | 508.06 | 12.47 | 32 | 33.85 | 49.75 | 1.22 |
| 16 | 21.06 | 621.96 | 15.27 | 33 | 35.61 | 98.70 | 2.42 |
| 17 | 21.66 | 259.70 | 6.38 | - | - | - | - |

In some embodiments of the present disclosure, the crystal form E has a differential scanning calorimetry (DSC) curve showing an endothermic peak with an onset at 268.6 ± 3°C.

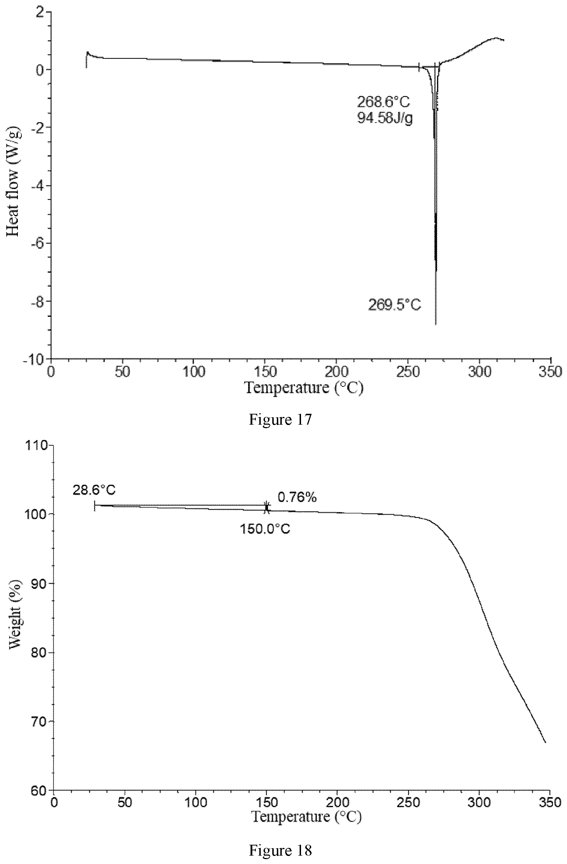

In some embodiments of the present disclosure, the crystal form E has a DSC pattern as shown in Figure 17.

In some embodiments of the present disclosure, the crystal form E has a DSC pattern basically as shown in Figure 17.

In some embodiments of the present disclosure, the crystal form E has a thermogravimetric analysis (TGA) curve with a weight loss of 0.76% at 150°C±3°C.

In some embodiments of the present disclosure, the crystal form E has a TGA pattern as shown in Figure 18.

In some embodiments of the present disclosure, the crystal form E has a TGA pattern basically as shown in Figure 18.

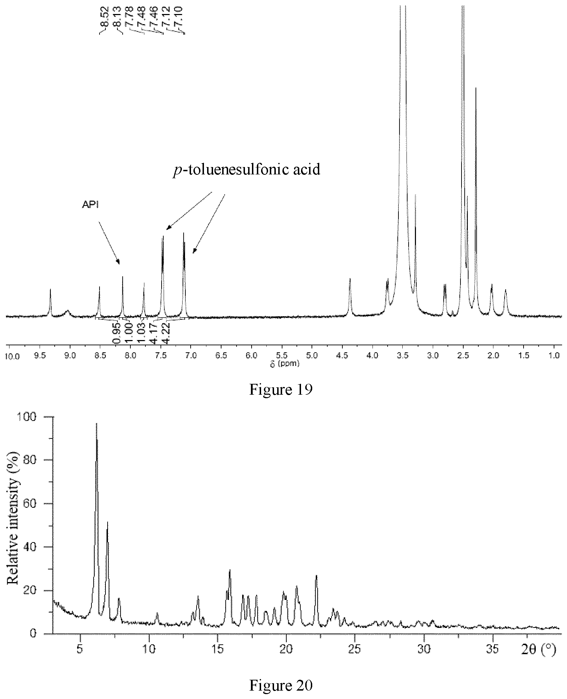

In some embodiments of the present disclosure, the crystal form E has a ¹H NMR pattern as shown in Figure 19.

In some embodiments of the present disclosure, the crystal form E has a ¹H NMR pattern basically as shown in Figure 19.

The present disclosure also provides a benzenesulfonate, an oxalate, a hydrobromide, a hydrochloride and a hydrate of the compound of formula (I). The structure of the benzenesulfonate is represented by formula (IV), the oxalate is represented by formula (V), the hydrobromide is represented by formula (VI), the hydrochloride is represented by formula (VII), and the hydrate is represented by formula (VIII), 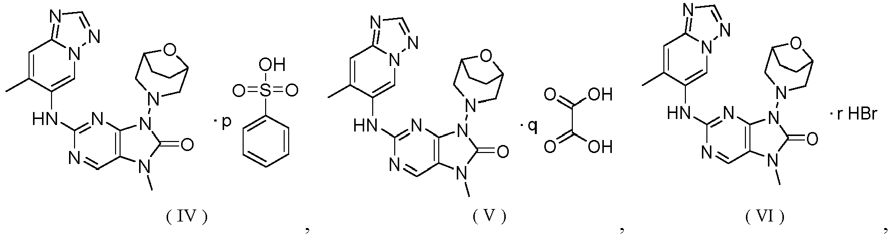 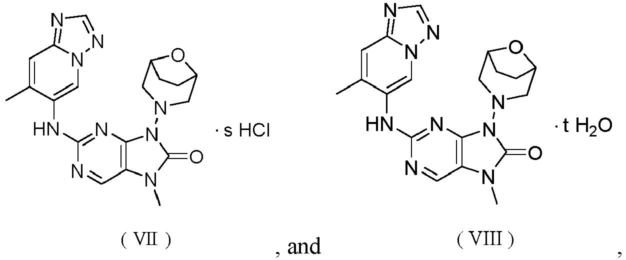 wherein p, q, and r are independently selected from 0.5 to 2.5, respectively; s and t are independently selected from 0.5 to 3.5, respectively.

In some embodiments of the present disclosure, p and r are independently selected from 0.8, 0.9, 1.0, 1.1, 1.2, 1.3, 1.4, 1.5, 1.6, 1.7, 1.8, 1.9, 2.0, 2.1 and 2.2, respectively; q is selected from 0.5, 0.6, 0.7, 0.8, 0.9, 1.0, 1.1, 1.2, 1.3, 1.4, 1.5, 1.6, 1.7, 1.8, 1.9, 2.0, 2.1 and 2.2; s and t are independently selected from 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, respectively.

In some embodiments of the present disclosure, p is selected from 1.0, 1.2 and 2.0.

In some embodiments of the present disclosure, q is selected from 0.6, 0.9 and 1.4.

In some embodiments of the present disclosure, r is selected from 1.0, 1.2 and 1.3.

In some embodiments of the present disclosure, s is selected from 0.8, 0.9, 1.0, 2.0, 2.2, 2.5 and 3.0.

In some embodiments of the present disclosure, t is selected from 0.8, 0.9, 1.0, 2.0, 2.2, 2.5 and 3.0.

In some embodiments of the present disclosure, the compound of formula (IV) is selected from a compound of formula (IV-1) and (IV-2), the compound of formula (V) is selected from a compound of formula (V-1), the compound of formula (VI) is selected from a compound of formula (VI-1), the compound of formula (VII) is selected from a compound of formula (VII-1), and the compound of formula (VIII) is selected from a compound of formula (VIII-1),

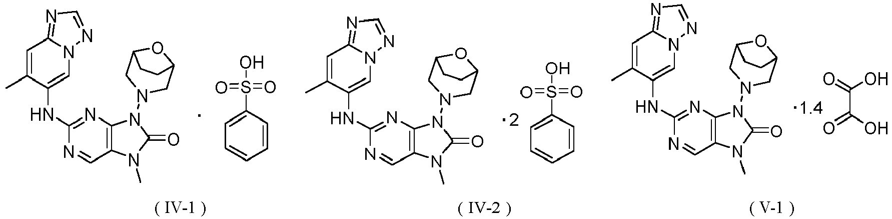

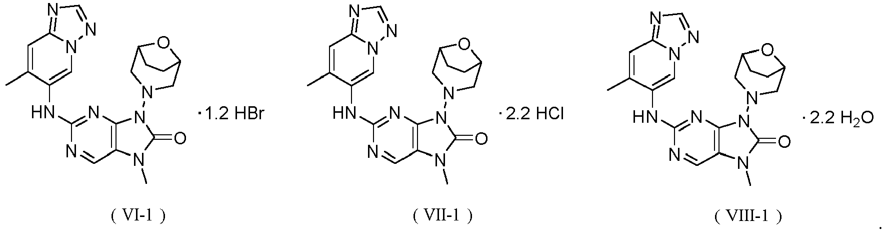

The present disclosure also provides a crystal form F of a compound of formula (IV-1), 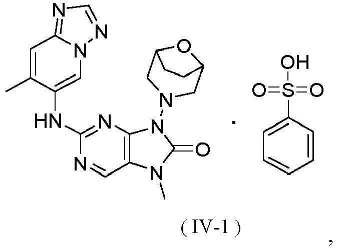 the crystal form F has an X-ray powder diffraction pattern of Cu Kα radiation comprising characteristic diffraction peaks at the following 20 angles: 6.21±0.20°, 6.97±0.20°, 13.58±0.20°, 15.90±0.20°, 17.81±0.20°, 19.76±0.20°, 20.73±0.20°, 22.17±0.20°.

In some embodiments of the present disclosure, the crystal form F has an X-ray powder diffraction pattern of Cu Kα radiation comprising characteristic diffraction peaks at the following 20 angles: 6.21±0.20°, 6.97±0.20°, 13.58±0.20°, and/or 7.84±0.20°, and/or 10.58±0.20°, and/or 13.17±0.20°, and/or 13.90±0.20°, and/or 15.65±0.20°, and/or 15.90±0.20°, and/or 16.83±0.20°, and/or 17.21±0.20°, and/or 17.81±0.20°, and/or 18.51±0.20°, and/or 19.12±0.20°, and/or 19.76±0.20°, and/or 19.99±0.20°, and/or 20.73±0.20°, and/or 22.17±0.20°, and/or 23.39±0.20°, and/or 23.69±0.20°, and/or 24.19±0.20°, and/or 24.83±0.20°, and/or 26.38±0.20°, and/or 28.30±0.20°, and/or 29.58±0.20°, and/or 30.62±0.20°, and/or 32.50±0.20°, and/or 34.06±0.20°, and/or 37.83±0.20°.

In some embodiments of the present disclosure, the crystal form F has an X-ray powder diffraction pattern of Cu Kα radiation comprising characteristic diffraction peaks at the following 20 angles: 6.21°, 6.97°, 7.84°, 10.58°, 13.17°, 13.58°, 13.90°, 15.65°, 15.90°, 16.83°, 17.21°, 17.81°, 18.51°, 19.12°, 19.76°, 19.99°, 20.73°, 22.17°, 23.39°, 23.69°, 24.19°, 24.83°, 26.38°, 28.30°, 29.58°, 30.62°, 32.50°, 34.06°, 37.83°.

In some embodiments of the present disclosure, the crystal form F has an XRPD pattern as shown in Figure 20.

In some embodiments of the present disclosure, the crystal form F has an XRPD pattern basically as shown in Figure 20.

In some embodiments of the present disclosure, the X-ray powder diffraction pattern of Cu Kα radiation of the crystal form F has peak positions and relative intensities of diffraction peaks as shown in the table below:

**Table 6 XRPD diffraction data of the crystal form F of the compound of formula (IV-1)**

| **No.** | **Diffraction angle 2θ** | **Peak height** | **Relative intensity (%)** | **No.** | **Diffraction angle 2θ** | **Peak height** | **Relative intensity (%)** |
|---|---|---|---|---|---|---|---|
| 1 | 6.21 | 3665.40 | 100.00 | 16 | 19.99 | 577.66 | 15.76 |
| 2 | 6.97 | 1888.74 | 51.53 | 17 | 20.73 | 801.72 | 21.87 |
| 3 | 7.84 | 423.24 | 11.55 | 18 | 22.17 | 1011.98 | 27.61 |
| 4 | 10.58 | 234.12 | 6.39 | 19 | 23.39 | 363.14 | 9.91 |
| 5 | 13.17 | 257.62 | 7.03 | 20 | 23.69 | 322.62 | 8.80 |
| 6 | 13.58 | 571.82 | 15.60 | 21 | 24.19 | 175.69 | 4.79 |
| 7 | 13.90 | 182.38 | 4.98 | 22 | 24.83 | 96.77 | 2.64 |
| 8 | 15.65 | 709.83 | 19.37 | 23 | 26.38 | 88.93 | 2.43 |
| 9 | 15.90 | 1099.04 | 29.98 | 24 | 28.30 | 106.35 | 2.90 |
| 10 | 16.83 | 617.16 | 16.84 | 25 | 29.58 | 118.23 | 3.23 |
| 11 | 17.21 | 599.16 | 16.35 | 26 | 30.62 | 140.20 | 3.82 |
| 12 | 17.81 | 619.20 | 16.89 | 27 | 32.50 | 44.17 | 1.21 |
| 13 | 18.51 | 289.19 | 7.89 | 28 | 34.06 | 52.79 | 1.44 |
| 14 | 19.12 | 385.70 | 10.52 | 29 | 37.83 | 30.88 | 0.84 |
| 15 | 19.76 | 677.49 | 18.48 | - | - | - | - |

In some embodiments of the present disclosure, the crystal form F has a differential scanning calorimetry (DSC) curve showing thermal signals with onsets at 216.9°C± 3°C, 219.6°C± 3°C and 240.7°C±3°C.

In some embodiments of the present disclosure, the crystal form F has a differential scanning calorimetry (DSC) curve showing endothermic peaks with onsets at 216.9°C± 3°C and 240.7°C ± 3°C.

In some embodiments of the present disclosure, the crystal form F has a differential scanning calorimetry (DSC) curve showing an endothermic peak with an onset at 219.6°C ± 3°C.

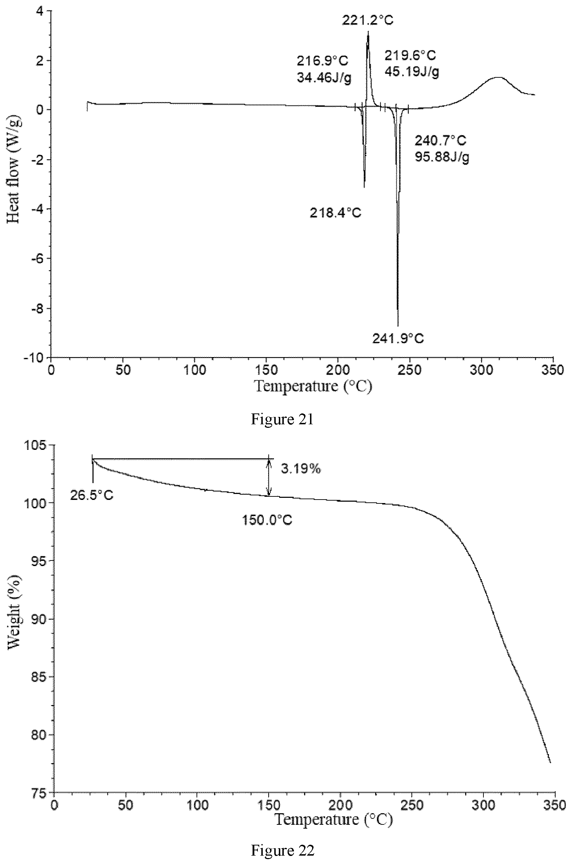

In some embodiments of the present disclosure, the crystal form F has a DSC pattern as shown in Figure 21.

In some embodiments of the present disclosure, the crystal form F has a DSC pattern basically as shown in Figure 21.

In some embodiments of the present disclosure, the crystal form F has a thermogravimetric analysis (TGA) curve with a weight loss of 3.19% at 150°C±3°C.

In some embodiments of the present disclosure, the crystal form F has a TGA pattern as shown in Figure 22.

In some embodiments of the present disclosure, the crystal form F has a TGA pattern basically as shown in Figure 22.

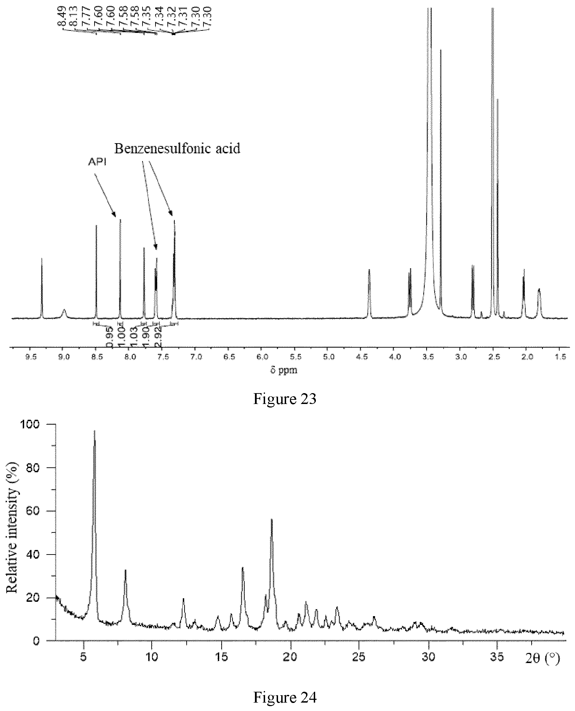

In some embodiments of the present disclosure, the crystal form F has a ¹H NMR pattern basically as shown in Figure 23.

In some embodiments of the present disclosure, the crystal form F has a ¹H NMR pattern basically as shown in Figure 23.

The present disclosure also provides a crystal form G of a compound of formula (IV-2), 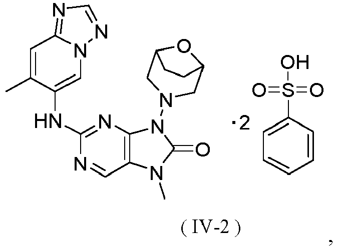 the crystal form G has an X-ray powder diffraction pattern of Cu Kα radiation comprising characteristic diffraction peaks at the following 20 angles: 5.83±0.20°, 8.06±0.20°, 12.27±0.20°, 16.56±0.20°, 18.67±0.20°.

In some embodiments of the present disclosure, the crystal form G has an X-ray powder diffraction pattern of Cu Kα radiation comprising characteristic diffraction peaks at the following 20 angles: 5.83±0.20°, 8.06±0.20°, 12.27±0.20°, and/or 13.06±0.20°, and/or 14.75±0.20°, and/or 15.72±0.20°, and/or 16.56±0.20°, and/or 18.23±0.20°, and/or 18.67±0.20°, and/or 19.69±0.20°, and/or 20.64±0.20°, and/or 21.11±0.20°, and/or 21.91±0.20°, and/or 22.57±0.20°, and/or 23.33±0.20°, and/or 24.25±0.20°, and/or 26.09±0.20°, and/or 29.00°.

In some embodiments of the present disclosure, the crystal form G has an X-ray powder diffraction pattern of Cu Kα radiation comprising characteristic diffraction peaks at the following 20 angles: 5.83°, 8.06°, 12.27°, 13.06°, 14.75°, 15.72°, 16.56°, 18.23°, 18.67°, 19.69°, 20.64°, 21.11°, 21.91°, 22.57°, 23.33°, 24.25°, 26.09°, 29.00°.

In some embodiments of the present disclosure, the crystal form G has an XRPD pattern as shown in Figure 24.

In some embodiments of the present disclosure, the crystal form G has an XRPD pattern basically as shown in Figure 24.

In some embodiments of the present disclosure, the X-ray powder diffraction pattern of Cu Kα radiation of the crystal form G has peak positions and relative intensities of diffraction peaks as shown in the table below:

**Table 7 XRPD diffraction data of the crystal form G of the compound of formula (IV-2)**

| **No.** | **Diffraction angle 2θ** | **Peak height** | **Relative intensity (%)** | **No.** | **Diffraction angle 2θ** | **Peak height** | **Relative intensity (%)** |
|---|---|---|---|---|---|---|---|
| 1 | 5.83 | 2976.45 | 100.00 | 10 | 19.69 | 130.64 | 4.39 |
| 2 | 8.06 | 875.80 | 29.42 | 11 | 20.64 | 250.49 | 8.42 |
| 3 | 12.27 | 470.26 | 15.80 | 12 | 21.11 | 451.88 | 15.18 |
| 4 | 13.06 | 116.89 | 3.93 | 13 | 21.91 | 319.36 | 10.73 |
| 5 | 14.75 | 213.26 | 7.16 | 14 | 22.57 | 222.20 | 7.47 |
| 6 | 15.72 | 240.47 | 8.08 | 15 | 23.33 | 359.20 | 12.07 |
| 7 | 16.56 | 993.57 | 33.38 | 16 | 24.25 | 122.90 | 4.13 |
| 8 | 18.23 | 554.14 | 18.62 | 17 | 26.09 | 212.77 | 7.15 |
| 9 | 18.67 | 1745.41 | 58.64 | 18 | 29.00 | 133.84 | 4.50 |

In some embodiments of the present disclosure, the crystal form G has a differential scanning calorimetry (DSC) curve showing endothermic peaks with onsets at 224.4°C± 3°C, 251.5°C± 3°C, and three thermal signal onsets at 233.7°C ± 3°C.

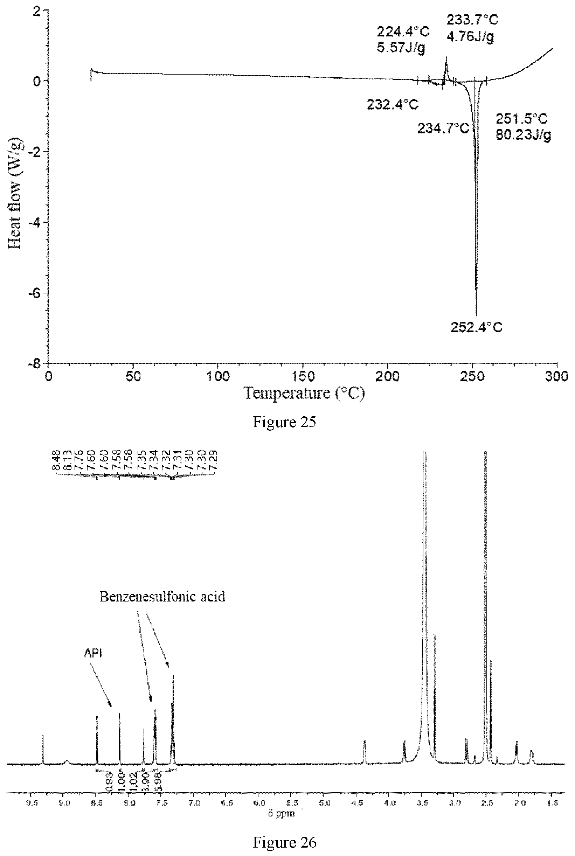

In some embodiments of the present disclosure, the crystal form G has a DSC pattern as shown in Figure 25.

In some embodiments of the present disclosure, the crystal form G has a DSC pattern basically as shown in Figure 25.

In some embodiments of the present disclosure, the crystal form G has a ¹H NMR pattern as shown in Figure 26.

In some embodiments of the present disclosure, the crystal form G has a ¹H NMR pattern basically as shown in Figure 26.

The present disclosure also provides a crystal form H of a compound of formula (V-1), 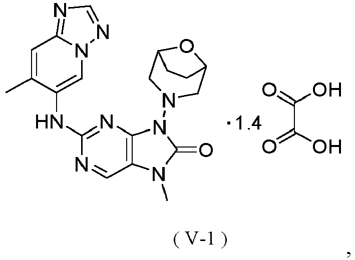 the crystal form H has an X-ray powder diffraction pattern of Cu Kα radiation comprising characteristic diffraction peaks at the following 20 angles: 4.31°, 8.52°, 11.28°, 12.75°, 13.32°, 14.52°, 15.4°, 16.48°, 17.19°, 18.32°, 18.87°, 19.09°, 19.78°, 20.4°, 21.28°, 25.4°, 26.99°, 27.34°, 29.22°, 29.93°, 30.69°, 35.02°, 36.16°.

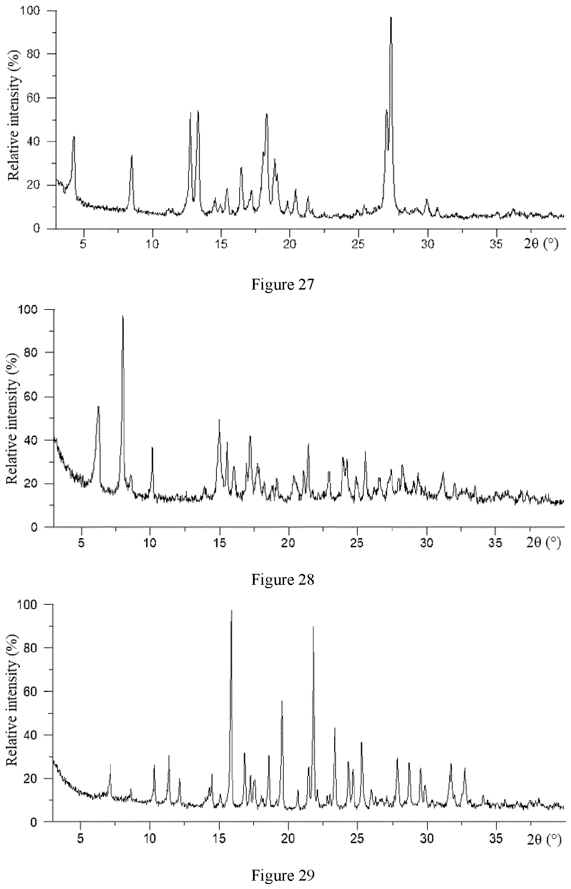

In some embodiments of the present disclosure, the crystal form H has an XRPD pattern as shown in Figure 27.

In some embodiments of the present disclosure, the crystal form H has an XRPD pattern basically as shown in Figure 27.

In some embodiments of the present disclosure, the X-ray powder diffraction pattern of Cu Kα radiation of the crystal form H has peak positions and relative intensities of diffraction peaks as shown in the table below:

**Table 8 XRPD diffraction data of the crystal form H of the compound of formula (V-1)**

| **No.** | **Diffraction angle 2θ** | **Peak height** | **Relative intensity (%)** | **No.** | **Diffraction angle 2θ** | **Peak height** | **Relative intensity (%)** |
|---|---|---|---|---|---|---|---|
| 1 | 4.31 | 807.33 | 29.30 | 13 | 19.78 | 217.17 | 7.88 |
| 2 | 8.52 | 796.80 | 28.91 | 14 | 20.40 | 293.58 | 10.65 |
| 3 | 11.28 | 43.53 | 1.58 | 15 | 21.28 | 276.77 | 10.04 |
| 4 | 12.75 | 1405.29 | 50.99 | 16 | 25.40 | 142.88 | 5.18 |
| 5 | 13.32 | 1400.98 | 50.84 | 17 | 26.99 | 1478.93 | 53.67 |
| 6 | 14.52 | 193.61 | 7.03 | 18 | 27.34 | 2755.77 | 100.00 |
| 7 | 15.40 | 360.38 | 13.08 | 19 | 29.22 | 94.43 | 3.43 |
| 8 | 16.48 | 678.63 | 24.63 | 20 | 29.93 | 231.77 | 8.41 |
| 9 | 17.19 | 336.12 | 12.20 | 21 | 30.69 | 104.88 | 3.81 |
| 10 | 18.32 | 1391.94 | 50.51 | 22 | 35.02 | 42.54 | 1.54 |
| 11 | 18.87 | 724.23 | 26.28 | 23 | 36.16 | 53.92 | 1.96 |
| 12 | 19.09 | 546.48 | 19.83 | - | - | - | - |

The present disclosure also provides a crystal form I of a compound of formula (VI-1), 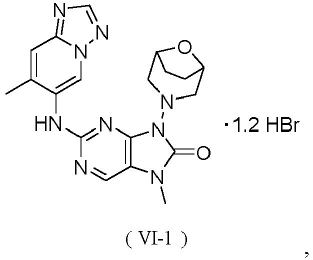 the crystal form I has an X-ray powder diffraction pattern of Cu Kα radiation comprising characteristic diffraction peaks at the following 20 angles: 6.27°, 8.02°, 8.59°, 10.14°, 13.90°, 14.99°, 15.56°, 16.07°, 16.95°, 17.20°, 17.82°, 19.16°, 20.39°, 21.10°, 21.44°, 22.94°, 23.92°, 24.26°, 24.91°, 25.57°, 26.58°, 27.42°, 28.26°, 29.39°, 31.19°, 32.02°, 33.52°.

In some embodiments of the present disclosure, the crystal form I has an XRPD pattern as shown in Figure 28.

In some embodiments of the present disclosure, the crystal form I has an XRPD pattern basically as shown in Figure 28.

In some embodiments of the present disclosure, the X-ray powder diffraction pattern of Cu Kα radiation of the crystal form I has peak positions and relative intensities of diffraction peaks as shown in the table below:

**Table 9 XRPD diffraction data of the crystal form I of the compound of formula (VI-1)**

| **No.** | **Diffraction angle 2θ** | **Peak height** | **Relative intensity (%)** | **No.** | **Diffraction angle 2θ** | **Peak height** | **Relative intensity (%)** |
|---|---|---|---|---|---|---|---|
| 1 | 6.27 | 626.52 | 45.11 | 15 | 21.44 | 445.48 | 32.07 |
| 2 | 8.02 | 1389.00 | 100.00 | 16 | 22.94 | 214.03 | 15.41 |
| 3 | 8.59 | 143.55 | 10.34 | 17 | 23.92 | 342.38 | 24.65 |
| 4 | 10.14 | 395.37 | 28.46 | 18 | 24.26 | 294.13 | 21.18 |
| 5 | 13.90 | 75.89 | 5.46 | 19 | 24.91 | 159.91 | 11.51 |
| 6 | 14.99 | 556.98 | 40.10 | 20 | 25.57 | 362.76 | 26.12 |
| 7 | 15.56 | 439.95 | 31.67 | 21 | 26.58 | 173.10 | 12.46 |
| 8 | 16.07 | 262.74 | 18.92 | 22 | 27.42 | 224.17 | 16.14 |
| 9 | 16.95 | 268.39 | 19.32 | 23 | 28.26 | 262.30 | 18.88 |
| 10 | 17.20 | 484.23 | 34.86 | 24 | 29.39 | 173.15 | 12.47 |
| 11 | 17.82 | 229.71 | 16.54 | 25 | 31.19 | 189.29 | 13.63 |
| 12 | 19.16 | 138.22 | 9.95 | 26 | 32.02 | 129.41 | 9.32 |
| 13 | 20.39 | 178.57 | 12.86 | 27 | 33.52 | 96.99 | 6.98 |
| 14 | 21.10 | 219.33 | 15.79 | - | - | - | - |

The present disclosure also provides a crystal form J of a compound of formula (VII-1), 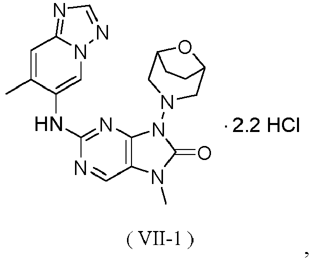 the crystal form J has an X-ray powder diffraction pattern of Cu Kα radiation comprising characteristic diffraction peaks at the following 20 angles: 7.14°, 8.58°, 10.33°, 11.36°, 12.15°, 14.47°, 15.08°, 15.86°, 16.85°, 17.25°, 17.58°, 18.08°, 18.58°, 19.54°, 20.69°, 21.44°, 21.81°, 22.10°, 23.35°, 24.33°, 24.66°, 25.29°, 25.96°, 27.88°, 28.71°, 29.53°, 29.84°, 31.74°, 32.71°, 34.04°, 37.79°.

In some embodiments of the present disclosure, the crystal form J has an XRPD pattern as shown in Figure 29.

In some embodiments of the present disclosure, the crystal form J has an XRPD pattern basically as shown in Figure 29.

In some embodiments of the present disclosure, the X-ray powder diffraction pattern of Cu Kα radiation of the crystal form J has peak positions and relative intensities of diffraction peaks as shown in the table below:

**Table 10 XRPD diffraction data of the crystal form J of the compound of formula (VII-1)**

| **No.** | **Diffraction angle 2θ** | **Peak height** | **Relative intensity (%)** | **No.** | **Diffraction angle 2θ** | **Peak height** | **Relative intensity (%)** |
|---|---|---|---|---|---|---|---|
| 1 | 7.14 | 366.08 | 16.78 | 17 | 21.81 | 1988.21 | 91.13 |
| 2 | 8.58 | 76.34 | 3.50 | 18 | 22.10 | 204.08 | 9.35 |
| 3 | 10.33 | 416.43 | 19.09 | 19 | 23.35 | 895.06 | 41.03 |
| 4 | 11.36 | 538.48 | 24.68 | 20 | 24.33 | 501.38 | 22.98 |
| 5 | 12.15 | 285.42 | 13.08 | 21 | 24.66 | 402.48 | 18.45 |
| 6 | 14.47 | 356.16 | 16.32 | 22 | 25.29 | 701.92 | 32.17 |
| 7 | 15.08 | 143.54 | 6.58 | 23 | 25.96 | 179.36 | 8.22 |
| 8 | 15.86 | 2181.72 | 100.00 | 24 | 27.88 | 527.68 | 24.19 |
| 9 | 16.85 | 614.50 | 28.17 | 25 | 28.71 | 502.40 | 23.03 |
| 10 | 17.25 | 344.72 | 15.80 | 26 | 29.53 | 438.88 | 20.12 |
| 11 | 17.58 | 304.31 | 13.95 | 27 | 29.84 | 239.02 | 10.96 |
| 12 | 18.08 | 90.69 | 4.16 | 28 | 31.74 | 487.14 | 22.33 |
| 13 | 18.58 | 603.24 | 27.65 | 29 | 32.71 | 421.26 | 19.31 |
| 14 | 19.54 | 1122.21 | 51.44 | 30 | 34.04 | 118.56 | 5.43 |
| 15 | 20.69 | 196.21 | 8.99 | 31 | 37.79 | 29.50 | 1.35 |
| 16 | 21.44 | 474.01 | 21.73 | - | - | - | - |

The present disclosure also provides a crystal form K of a compound of formula (VIII-1), 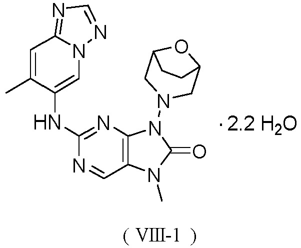 the crystal form K has an X-ray powder diffraction pattern of Cu Kα radiation comprising characteristic diffraction peaks at the following 20 angles: 6.99°, 7.34°, 8.33°, 9.22°, 9.92°, 10.56°, 10.97°, 13.35°, 13.96°, 14.60°, 15.13°, 15.83°, 16.36°, 16.91°, 18.48°, 19.58°, 20.62°, 20.99°, 22.22°, 22.67°, 23.78°, 25.77°, 26.19°, 26.84°, 27.46°, 31.21°, 37.22°.

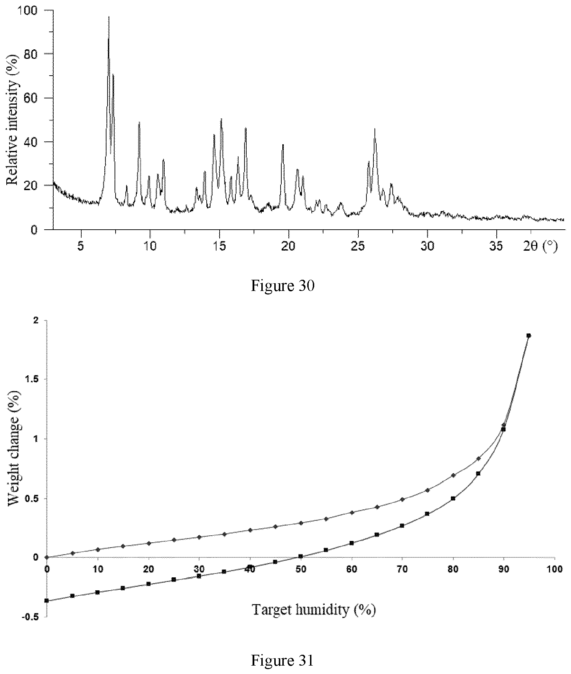

In some embodiments of the present disclosure, the crystal form K has an XRPD pattern as shown in Figure 30.

In some embodiments of the present disclosure, the crystal form K has an XRPD pattern basically as shown in Figure 30.

In some embodiments of the present disclosure, the X-ray powder diffraction pattern of Cu Kα radiation of the crystal form K has peak positions and relative intensities of diffraction peaks as shown in the table below:

**Table 11 XRPD diffraction data of the crystal form K of the compound of formula (VIII-1)**

| **No.** | **Diffraction angle 2θ** | **Peak height** | **Relative intensity (%)** | **No.** | **Diffraction angle 2θ** | **Peak height** | **Relative intensity (%)** |
|---|---|---|---|---|---|---|---|
| 1 | 6.99 | 2948.60 | 100.00 | 15 | 18.48 | 138.53 | 4.70 |
| 2 | 7.34 | 2087.30 | 70.79 | 16 | 19.58 | 1108.68 | 37.60 |
| 3 | 8.33 | 257.73 | 8.74 | 17 | 20.62 | 714.58 | 24.23 |
| 4 | 9.22 | 1358.47 | 46.07 | 18 | 20.99 | 600.93 | 20.38 |
| 5 | 9.92 | 488.45 | 16.57 | 19 | 22.22 | 214.80 | 7.28 |
| 6 | 10.56 | 565.01 | 19.16 | 20 | 22.67 | 154.95 | 5.26 |
| 7 | 10.97 | 804.48 | 27.28 | 21 | 23.78 | 184.49 | 6.26 |
| 8 | 13.35 | 338.84 | 11.49 | 22 | 25.77 | 836.23 | 28.36 |
| 9 | 13.96 | 655.05 | 22.22 | 23 | 26.19 | 1382.89 | 46.90 |
| 10 | 14.60 | 1236.10 | 41.92 | 24 | 26.84 | 412.81 | 14.00 |
| 11 | 15.13 | 1451.49 | 49.23 | 25 | 27.46 | 505.00 | 17.13 |
| 12 | 15.83 | 532.35 | 18.05 | 26 | 31.21 | 44.76 | 1.52 |
| 13 | 16.36 | 874.04 | 29.64 | 27 | 37.22 | 42.01 | 1.42 |
| 14 | 16.91 | 1374.26 | 46.61 | - | - | - | - |

The present disclosure also provides a use of the mesylate, the *p*-toluenesulfonate, the crystal form B, the crystal form C or the crystal form A of the compound in the preparation of a DNA-PK inhibitor-related medicament.

The present disclosure also provides a use of the methanesulfonate, the *p-*toluenesulfonate, the benzenesulfonate, the oxalate, the hydrobromide, the hydrochloride, the hydrate, the crystal form A, the crystal form B, the crystal form C, the crystal form D, the crystal form E, the crystal form F, the crystal form G, the crystal form H, the crystal form I, the crystal form J or the crystal form K of the compound in the preparation of a DNA-PK inhibitor-related medicament.

### Technical effect

As a DNA-PK inhibitor, the compound of the present disclosure exhibits excellent DNA-PK kinase inhibitory activity. The PK results demonstrate that the compound of the present disclosure possesses a longer half-life, a higher drug exposure and excellent pharmacokinetics *in vivo*, which is a good molecule that can be developed for oral administration.

### Definition and description

Unless otherwise specified, the following terms and phrases when used herein have the following meanings. A specific phrase or term should not be considered indefinite or unclear in the absence of a particular definition, but should be understood in the ordinary sense. When a trading name appears herein, it is intended to refer to its corresponding commodity or active ingredient thereof.

The intermediate compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The term "pharmaceutically acceptable salt" refers to a salt of the compound that is prepared by reacting the compound with a relatively non-toxic acid or base; based on the nature of the compound of formula (I) of the present disclosure, the salt is preferably prepared with a relatively non-toxic acid. The acid addition salt can be obtained by contacting the compound with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, naphthalenedisulfonic acid, citric acid, tartaric acid, and methanesulfonic acid, etc.

The compound of formula (I) and the pharmaceutically acceptable salt thereof in the present disclosure include, but are not limited to, hydrobromide, methanesulfonate, oxalate, *p-*toluenesulfonic acid, and a solvate thereof; wherein a hydrate is an embodiment of a solvate. In the pharmaceutically acceptable salt or solvate of the compound of formula (I) in the present disclosure, the amount of the combined acid or solvent is represented by the molar ratio of the acid or solvent to the compound of formula (I). For example, the compound of formula (I) · 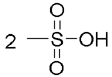 represents the addition salt of the compound of formula (I) and methanesulfonic acid. In the acid addition salt, the molar ratio of methanesulfonic acid to the compound of formula (I) is 2:1.

The chemical reactions of the specific embodiments of the present disclosure are completed in a suitable solvent, and the solvent must be suitable for the chemical changes of the present disclosure and the reagents and materials required therefor. In order to obtain the compound of the present disclosure, those skilled in the art sometimes need to modify or select the synthesis steps or reaction procedures based on the existing embodiments.

Unless otherwise specified, in differential scanning calorimetry curves for the compound of the present disclosure, an upward direction indicates heat release.

The present disclosure is described in detail by the examples below, but it does not mean that there are any adverse restrictions on the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: ϕ/ω scan, and after collecting the relevant data, the crystal structure is further analyzed by direct method (Shelxs97), so that the absolute configuration can be confirmed.

The following abbreviations are used in the present disclosure: ACN represents acetonitrile; DMSO represents dimethyl sulfoxide. N₂: nitrogen; RH: relative humidity; mL: milliliter; L: liter; min: minute; °C: degree Celsius; µm: micrometers; mm: millimeter; µL: microliter; moL/L: mole per liter; mg: milligram; s: second; nm: nanometer; MPa: megapascal; lux: lux; µw/cm²: microwatt per square centimeter; h: hour; Kg: kilogram; nM: nanomole, rpm: rotational speed; XRPD represents X-ray powder diffraction; DSC represents differential scanning calorimetry; TGA represents thermogravimetric analysis; ¹H NMR represents Nuclear Magnetic Resonance Hydrogen Spectrum.

The compounds of the present disclosure are named according to the conventional naming principles in the art or by ChemDraw^{®} software, the commercially available compounds use the supplier catalog names, and all solvents used in the present disclosure are commercially available.

### Instrument and analysis method of the present disclosure

### 1.1 X-ray powder diffractometer (XRPD) method

Test method: Approximately 10 mg of sample was used for XRPD test.

Detailed XRPD instrument information and parameters are shown in the table below:

**Table 12 XRPD instrument information and parameters**

| | |
|---|---|
| Instrument model | PANalytical X'Pert³ X-ray powder diffractometer |
| X-ray | Cu, kα, Kα1 (Å): 1.540598; Kα2 (Å); 1.544426 |
| Kα2/Kα1 intensity ratio | 0.50 |
| X-ray tube setting | Tube voltage: 45 kV; Tube current: 40 mA |
| Divergence slit | 1/8 degree |
| Scan mode | Continuous |
| Scan range | 3-40 deg |
| Step size | 0.0263 deg |
| Scan time per step | 46.665 seconds |

### 1.2 Differential Scanning Calorimeter (DSC) and Thermal Gravimetric Analyzer (TGA)

Instrument model: Discovery DSC 2500 differential scanning calorimeter and Discovery 5500 TGA thermal gravimetric analyzer. The detailed parameters are as shown in the table below:

**Table 13 DSC and TGA instrument parameters**

| **Parameters** | **TGA** | **DSC** |
|---|---|---|
| Method | Linear heating | Linear heating |
| Sample tray | Aluminum tray, uncapped | Aluminum tray, capped |
| Temperature range | Room temperature - setting endpoint temperature | 25°C - setting endpoint temperature |
| Scan rate (°C/minutes) | 10 | 10 |
| Protective gas | Nitrogen | Nitrogen |

### 1.3 Hydrogen Nuclear Magnetic Resonance Spectrum (¹H NMR)

Instrument: Bruker 400M NMR Spectrometer, deuterated solvent: DMSO-*d*6.

### 1.4 Dynamic Vapor Sorption (DVS) method

Instrument model: SMS DVS intrinsic dynamic moisture sorption analyzer
Test conditions: a sample (about 10 mg) is taken and placed in the DVS sample tray for testing.

The detailed DVS parameters are as follows:
Temperature: 25°C
Equilibrium: dm/dt=0.01%/min
RH (%) test increment: 5% RH
RH (%) test increment range: 0% to 95% to 0%

The evaluation and classifications of hygroscopicity are shown as follows:
Sufficient moisture is absorbed to produce a fluid: Deliquescence; ΔW% ≥ 15%: High hygroscopicity; 15% > ΔW% ≥ 2%: Hygroscopicity; 2%> ΔW%≥ 0.2%: Slight hygroscopicity; ΔW% < 0.2%: No hygroscopicity or almost no hygroscopicity: ΔW% indicates hygroscopic weight gain of the sample at 25 ± 1°C and 80 ± 2% RH.

### Ultra-high performance liquid chromatography/ion chromatography (UPLC/IC) instrument

The acid-base molar ratio test in the experiment is tested by ultra-high performance liquid chromatography and ion chromatography. The analysis conditions are shown in Table 14 and Table 15.

**Table 14 Test conditions of ultra-high performance liquid chromatography**

| **Liquid chromatography** | **Waters H-Class UPLC** | |
|---|---|---|
| Chromatography column | Waters BEH C18 2.1 mm/50 mm/1.7 µm | |
| Mobile phase | Phase A: 0.05% trifluoroacetic acid in aqueous solution; Phase B: 0.05% trifluoroacetic acid in acetonitrile solution | |
| Gradient | Time (minute) | %B |
| | 0 | 10 |
| | 1.00 | 10 |
| | 5.00 | 80 |
| | 6.00 | 80 |
| | 6.10 | 10 |
| | 8.00 | 10 |
| Operation time | 8.0 minutes | |
| Flow rate of mobile phase | 0.5 mL/minute | |
| Injection volume | 2 microliters | |
| Detection wavelength | 210 nanometers | |
| Column temperature | 25°C | |
| Diluter | Ethanol/water (1:1, volume ratio) | |

**Table 15 Test conditions of ion chromatography**

| **Ion chromatography** | **ThermoFisher ICS-1100** |
|---|---|
| Chromatography column | IonPac AS 18 Analytical Column, 4 × 250 mm |
| Mobile phase | 25 mM NaOH |
| Operation time | 7 minutes (chloride ion); 18 minutes (bromide ion) |
| Flow rate | 1.0 mL/minute |
| Injection volume | 25 microliters |
| Electrical current | 80 milliamperes |
| Column temperature | 35°C |

### BRIEF DESCRIPTION OF THE DRAWINGS

Figure 1 is an XRPD pattern of the crystal form A of the compound of formula (I).
Figure 2 is a DSC pattern of the crystal form A of the compound of formula (I).
Figure 3 is a TGA pattern of the crystal form A of the compound of formula (I).
Figure 4 is an XRPD pattern of the crystal form B of compound of formula (II-1).
Figure 5 is a DSC pattern of the crystal form B of compound of formula (II-1).
Figure 6 is a TGA pattern of the crystal form B of compound of formula (II-1).
Figure 7 is a ¹H NMR pattern of the crystal form B of compound of formula (II-1).
Figure 8 is an XRPD pattern of the crystal form C of compound of formula (III-1).
Figure 9 is a DSC pattern of the crystal form C of compound of formula (III-1).
Figure 10 is a TGA pattern of the crystal form C of compound of formula (III-1).
Figure 11 is a ¹H NMR pattern of the crystal form C of compound of formula (III-1).
Figure 12 is an XRPD pattern of the crystal form D of compound of formula (II-2).
Figure 13 is a DSC pattern of the crystal form D of compound of formula (II-2).
Figure 14 is a TGA pattern of the crystal form D of compound of formula (II-2).
Figure 15 is a ¹H NMR pattern of the crystal form D of compound of formula (II-2).
Figure 16 is an XRPD pattern of the crystal form E of compound of formula (III-2).
Figure 17 is a DSC pattern of the crystal form E of compound of formula (III-2).
Figure 18 is a TGA pattern of the crystal form E of compound of formula (III-2).
Figure 19 is a ¹H NMR pattern of the crystal form E of compound of formula (III-2).
Figure 20 is an XRPD pattern of the crystal form F of compound of formula (IV-1).
Figure 21 is a DSC pattern of the crystal form F of compound of formula (IV-1).
Figure 22 is a TGA pattern of the crystal form F of compound of formula (IV-1).
Figure 23 is a ¹H NMR pattern of the crystal form F of compound of formula (IV-1).
Figure 24 is an XRPD pattern of the crystal form G of compound of formula (IV-2).
Figure 25 is a DSC pattern of the crystal form G of compound of formula (IV-2).
Figure 26 is a ¹H NMR pattern of the crystal form G of compound of formula (IV-2).
Figure 27 is an XRPD pattern of the crystal form H of compound of formula (V-1).
Figure 28 is an XRPD pattern of the crystal form I of compound of formula (VI-1).
Figure 29 is an XRPD pattern of the crystal form J of compound of formula (VII-1).
Figure 30 is an XRPD pattern of the crystal form K of compound of formula (VIII-1).
Figure 31 is a DVS pattern of the crystal form B of compound of formula (II-1).
Figure 32 is photographs of the tumor on day 21.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for one skilled in the art, it is obvious to make various modifications and improvements to the examples of the present disclosure without departing from the spirit and scope of the present disclosure.

### Example 1: Preparation of a compound of formula (I)

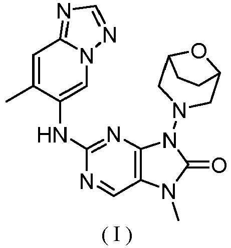

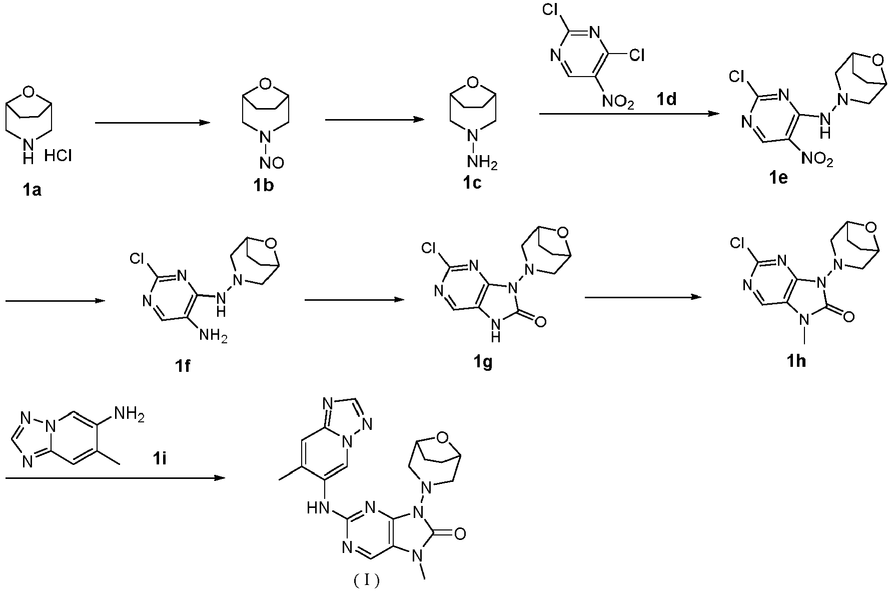

### Step 1

To a mixed solution of acetic acid (50 mL) and water (18 mL) of compound **1a** (4.49 g, 30 mmol, 1 *eq*) was slowly added a solution of sodium nitrite (2.28 g, 33 mmol, 1.1 *eq*) in water (4.5 mL) at 0°C, and after the addition, the reaction solution was reacted at 20°C for 3 hours. After the reaction was complete, the reaction solution was diluted with water (50 mL), and extracted with 150 mL of ethyl acetate (50 mL * 3). The organic phase was washed with saturated brine (30 mL), dried over anhydrous sodium sulfate, and filtered. The filtrate was concentrated under reduced pressure and purified by silica gel column chromatography (ethyl acetate: petroleum ether = 0:1 to 1:1) to obtain compound **1b.** MS: *m*/*z.* 143.0 [M+H]⁺.

### Step 2

To a solution of compound **1b** (3.70 g, 26 mmol, 1 *eq*) in methanol (20 mL) were subsequently added zinc powder (6.80 g, 104 mmol, 4 *eq*) and acetic acid (20 mL) at 0°C, and after the addition, the reaction solution was reacted at 20°C for 4 hours. After the reaction was completed, the reaction solution was directly filtered through diatomite and washed with ethyl acetate (200 mL). The filtrate was concentrated under reduced pressure to obtain a crude product of compound **1c.**

### Step 3

To a solution of compound **1d** (10.09 g, 52 mmol, 2 *eq*) in dioxane (150 mL) were subsequently added compound **1c** (4.89 g, 26 mmol, 1 *eq*) and triethylamine (13.15 g, 130 mmol, 5 *eq*, 18.09 mL) at 0°C, and after the addition, the reaction solution was reacted at 20°C for 5 hours. After the reaction was complete, the reaction solution was diluted with water (100 mL), and extracted with 300 mL of ethyl acetate (100 mL * 3). The organic phase was washed with saturated brine (50 mL), dried over anhydrous sodium sulfate, and filtered. The filtrate was concentrated under reduced pressure and purified by column chromatography (ethyl acetate: petroleum ether = 0:1 to 1:1) to obtain compound **1e.** MS: *m*/*z* 285.9 [M+H]⁺.

¹H NMR (400 MHz, CDCl₃) δ ppm 9.05 (s, 1H), 8.97 (br s, 1H), 4.43 (br dd, *J*=4.44, 2.06 Hz, 2H), 2.94-3.06 (m, 4H), 2.19-2.28 (m, 2H), 1.90-2.03 (m, 2H).

### Step 4

To a solution of ethanol (120 mL) and water (30 mL) of compound **1e** (2.43 g, 8.5 mmol, 1 *eq*) were subsequently added iron powder (2.37 g, 42.5 mmol, 5 *eq*) and ammonium chloride (2.27 g, 42.5 mmol, 5 *eq*), and after the addition, the reaction solution was reacted at 75°C for 3 hours. After the reaction was completed, the reaction solution was cooled to room temperature, diluted with ethyl acetate (200 mL), filtered through diatomite, and concentrated under reduced pressure to obtain crude compound **1f.** MS: *m*/*z* 256.0 [M+H]⁺.

### Step 5

To a solution of compound **1f** (2.17 g, 8.5 mmol, 1 *eq*) in acetonitrile (30 mL) was added *N,N'*-carbonyldiimidazole (2.76 g, 17 mmol, 2 *eq*), and after the addition, the reaction solution was reacted at 80°C for 2 hours. After the reaction was completed, the reaction solution was concentrated under reduced pressure and purified by column chromatography (ethyl acetate: petroleum ether = 0:1 to 1:0) to obtain compound **1g.** MS: m/z 281.9 [M+H]+.

¹H NMR (400 MHz, DMSO-*d*₆) δ ppm 11.61 (br s, 1H), 8.12 (s, 1H), 4.38 (brd, *J*=2.01 Hz, 2H), 3.73 (dd, *J*=9.91, 1.63 Hz, 2H), 2.81 (d, *J*=9.54 Hz, 2H), 1.99-2.09 (m, 2H), 1.78-1.87 (m, 2H).

### Step 6

To a solution of compound **1g** (1.24 g, 4.4 mmol, 1 *eq*) in *N,N*-dimethylformamide (40 mL) were subsequently added cesium carbonate (2.15 g, 6.6 mmol, 1.5 *eq*) and methyl iodide (780 mg, 5.5 mmol, 1.25 *eq*), and after the addition, the reaction solution was reacted at 21°C for 4 hours. After the reaction was complete, the reaction solution was diluted with water (100 mL), and extracted with 180 mL of ethyl acetate (60 mL * 3). The organic phase was washed with saturated brine (50 mL), dried over anhydrous sodium sulfate, and filtered. The filtrate was concentrated under reduced pressure and purified by column chromatography (ethyl acetate: petroleum ether = 0:1 to 4:1) to obtain compound **1h.** MS: *m*/*z* 295.9 [M+H]⁺.

¹H NMR (400 MHz, CDCl₃) δ ppm 7.98-8.05 (m, 1H), 4.45 (br d, *J*=2.25 Hz, 2H), 3.99 (dd, *J*=9.69, 1.81 Hz, 2H), 3.41 (s, 3H), 2.80 (br d, *J*=9.51 Hz, 2H), 2.23-2.31 (m, 2H), 1.94-2.04 (m, 2H).

### Step 7

Compound **1h** (502.7 mg, 1.7 mmol, 1 *eq*), compound **1i** (201.5 mg, 1.36 mmol, 0.8 *eq*), methanesulfonic acid (2-dicyclohexylphosphino-3,6-dimethoxy-2',4',6'-triisopropyl-1,1'-biphenyl)(2-amino-1,1'-biphen-2-yl)palladium(II) (231.2 mg, 255 µmol, 0.15 *eq*), and cesium carbonate (830.8 mg, 2.55 mmol, 1.5 *eq*) were placed in a reaction flask, and carried out three nitrogen replacements, then the mixture was added with anhydrous dioxane (30 mL), and reacted at 100°C for 3 hours. After the reaction was complete, the reaction solution was filtered through diatomite and concentrated under reduced pressure to obtain a crude product, which was then purified by column chromatography (methanol/dichloromethane, methanol ratio: 0% to 10%), slurried and stirred (dichloromethane/ethyl acetate: 1.5 mL/3 mL, 25°C, 15 minutes) to obtain a compound of formula (I). MS: *m*/*z* 408.2 [M+H]⁺.

¹H NMR (400 MHz, CDCl₃) δ ppm 9.87 (s, 1H), 8.26 (s, 1H), 7.91 (s, 1H), 7.57 (s, 1H), 6.76 (s, 1H), 4.48 (br d, *J*=2.25 Hz, 2H), 4.04 (dd, *J*=9.76, 1.88 Hz, 2H), 3.40 (s, 3H), 2.85 (d, *J*=9.51 Hz, 2H), 2.53 (s, 3H) 2.29-2.37 (m, 2H), 2.00-2.10 (m, 2H).

### Example 2: Preparation of a crystal form K of a compound of formula (VIII-1)

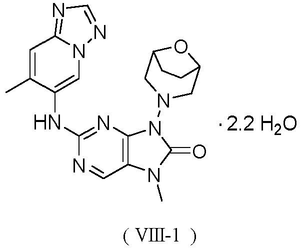

To the compound of formula (I) (44 g) was added deionized water (200 mL) at 25°C, and the mixture was stirred at 25°C for 5 hours, filtered, and the filter cake was dried under vacuum at 55°C for 18 hours to obtain a crystal form K of a compound of formula (VIII-1). The XRPD pattern is basically as shown in Figure 30.

### Example 3: Preparation of a crystal form A of a compound of formula (I)

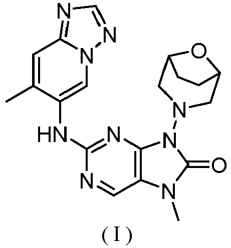

Method 1: About 20 mg of a solid of the crystal form K of the compound of formula (VIII-1) was weighed, and slurried and stirred with acetone (0.5 mL) at room temperature for 6 days, and then dried open at room temperature (40% to 60% RH/19°C to 21°C) over overnight to obtain the crystal form A of the compound of formula (I).

Method 2: To the crystal form K (74 g) of the compound of formula (VIII-1) was added 1500 mL of acetonitrile, and after the addition, the mixture was stirred at 60°C for 24 hours. The mixture was cooled to room temperature and filtered, and the filter cake was dried under vacuum at 25°C for 3 hours to obtain a crystal form A of a compound of formula (I).

The XRPD pattern is basically as shown in Figure 1, the DSC pattern is basically as shown in Figure 2, and the TGA pattern is basically as shown in Figure 3.

### Example 4: Preparation of a crystal form B of a compound of formula (II-1)

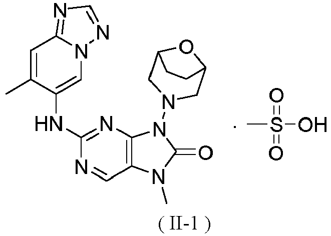

Method 1: About 20 mg (1 *eq*) of the crystal form A of the compound of formula (VIII-1) and methanesulfonic acid (1 *eq*) were weighed and added to ethanol/water (19:1, v/v, 0.5 mL), and the suspension was stirred at room temperature for 3 days. The mixture was centrifuged, and the solid was collected and dried under vacuum at room temperature for 4 hours to obtain a crystal form B of a compound of formula (II-1).

Method 2: The crystal form K of the compound of formula (VIII-1) (2 g, 4.90 mmol, 1 *eq*) was dissolved with acetonitrile (19 mL) at 25°C, and a solution of methanesulfonic acid (476.6 mg, 4.96 mmol, 1.01 *eq*) in acetonitrile (1 mL) was slowly added dropwise. After the addition, the mixture was stirred at 25°C for 16 hours, filtered, and the filter cake was washed with *n*-heptane (20 mL * 2) and dried under vacuum at 25°C for 1 hour to obtain a crystal form B of a compound of formula (II-1).

Method 3: The crystal form A of the compound of formula (I) (30 g, 73.63 mmol, 1 *eq*) was diluted with DMSO (300 mL) at 25°C, heated to 60°C, and stirred to dissolve. Methanesulfonic acid (3.54 g, 36.82 mmol, 0.5 *eq*) was added dropwise to the reaction solution and stirred at 60°C for 1 hour. Methanesulfonic acid (3.89 g, 40.50 mmol, 0.55 *eq*) was added dropwise to the reaction solution and stirred at 60°C for 12 hours. Methyl *tert*-butyl ether (450 mL) was added dropwise to the reaction solution and stirred at 60°C for another 1 hour. The reaction solution was then slowly cooled to 25°C, filtered, and the filter cake was washed with methyl *tert*-butyl ether (100 mL * 3) and dried under vacuum at 25°C for 1 hour to obtain a crystal form B of a compound of formula (II-1).

¹H NMR shows that the methanesulfonic acid and the compound of formula (I) has a molar ratio of 1. The XRPD pattern is basically as shown in Figure 4, the DSC pattern is basically as shown in Figure 5, the TGA pattern is basically as shown in Figure 6, and the ¹H NMR pattern is basically as shown in Figure 7.

### Example 5: Preparation of a crystal form D of a compound of formula (II-2)

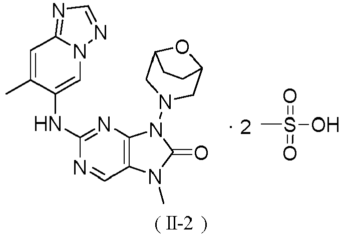

About 20 mg (1 *eq*) of the crystal form K of the compound of formula (VIII-1) and methanesulfonic acid (2 *eq*) were weighed and added to acetone (0.5 mL), and the mixture was stirred at room temperature for 2 days. The mixture was centrifuged, and the solid was collected and dried under vacuum at room temperature for 3 hours to obtain a crystal form D of a compound of formula (II-2). ¹H NMR shows that the methanesulfonic acid and the compound of formula (I) has a molar ratio of 2. The XRPD pattern is basically as shown in Figure 12, the DSC pattern is basically as shown in Figure 13, the TGA pattern is basically as shown in Figure 14, and the ¹H NMR pattern is basically as shown in Figure 15.

### Example 6: Preparation of a crystal form C of a compound of formula (III-1)

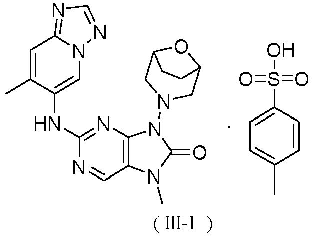

About 20 mg (1 *eq*) of the crystal form K of the compound of formula (VIII-1) and *p-*toluenesulfonic acid (1 *eq*) were weighed and added to tetrahydrofuran (0.5 mL), and the suspension was stirred at room temperature for 2 days. The mixture was centrifuged, and the solid was collected and dried under vacuum at room temperature for 3 hours to obtain a crystal form C of a compound of formula (III-1). ¹H NMR shows that the methanesulfonic acid and the compound of formula (I) has a molar ratio of 1. The XRPD pattern is basically as shown in Figure 8, the DSC pattern is basically as shown in Figure 9, the TGA pattern is basically as shown in Figure 10, and the ¹H NMR pattern is basically as shown in Figure 11.

### Example 7: Preparation of a crystal form E of a compound of formula (III-2)

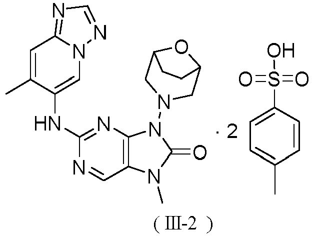

About 20 mg (1 *eq*) of the crystal form K of the compound of formula (VIII-1) and p-toluenesulfonic acid (2 *eq*) were weighed and added to acetone (0.5 mL), and the suspension was stirred at room temperature for 2 days. The mixture was centrifuged, and the solid was collected and dried under vacuum at room temperature for 3 hours to obtain a crystal form E of a compound of formula (III-2). ¹H NMR shows that the methanesulfonic acid and the compound of formula (I) has a molar ratio of 2. The XRPD pattern is basically as shown in Figure 15, the DSC pattern is basically as shown in Figure 17, the TGA pattern is basically as shown in Figure 18, and the ¹H NMR pattern is basically as shown in Figure 19.

### Example 8: Preparation of a crystal form F of a compound of formula (IV-1)

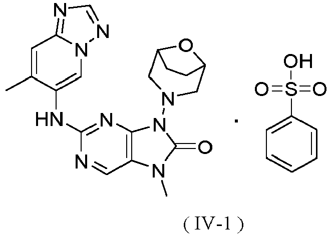

About 20 mg (1 *eq*) of the crystal form K of the compound of formula (VIII-1) and benzenesulfonic acid (1 *eq*) were weighed and added to ethanol/water (19:1, v/v, 0.5 mL), and the suspension was stirred at room temperature for 2 days. The mixture was centrifuged, and the solid was collected and dried under vacuum at room temperature for 3 hours to obtain a crystal form F of a compound of formula (IV-1). ¹H NMR shows that the methanesulfonic acid and the compound of formula (I) has a molar ratio of 1. The XRPD pattern is basically as shown in Figure 20, the DSC pattern is basically as shown in Figure 21, the TGA pattern is basically as shown in Figure 22, and the ¹H NMR pattern is basically as shown in Figure 23.

### Example 9: Preparation of a crystal form G of a compound of formula (IV-2)

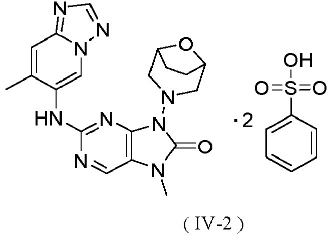

About 20 mg (1 *eq*) of the crystal form K of the compound of formula (VIII-1) and benzenesulfonic acid (2 *eq*) were weighed and added to acetone (0.5 mL), and the suspension was stirred at room temperature for 2 days. The mixture was centrifuged, and the solid was collected and dried under vacuum at room temperature for 3 hours to obtain a crystal form G of a compound of formula (IV-2). ¹H NMR shows that the methanesulfonic acid and the compound of formula (I) has a molar ratio of 2. The XRPD pattern is basically as shown in Figure 24, the DSC pattern is basically as shown in Figure 25, and the ¹H NMR pattern is basically as shown in Figure 26.

### Example 10: Preparation of a crystal form H of a compound of formula (V-1)

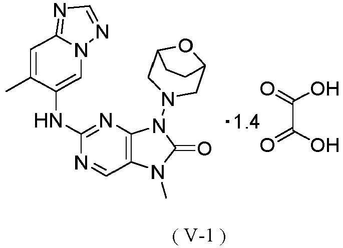

About 20 mg (1 *eq*) of the crystal form K of the compound of formula (VIII-1) and oxalic acid (2 *eq*) were weighed and added to acetone (0.5 mL), and the suspension was stirred at room temperature for 2 days. The mixture was centrifuged, and the solid was collected and dried under vacuum at room temperature for 3 hours to obtain a crystal form H of a compound of formula (V-1). The combination of high-performance liquid chromatography and ion chromatography (UPLC/IC) shows an acid-base molar ratio of 1.4, and the XRPD pattern is basically as shown in Figure 27.

### Example 11: Preparation of a crystal form I of a compound of formula (VI-1)

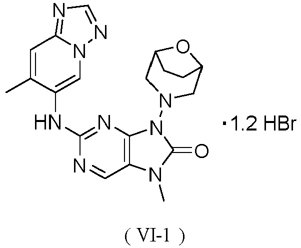

About 20 mg (1 *eq*) of the crystal form K of the compound of formula (VIII-1) and hydrobromic acid (1 *eq*) were weighed and added to ethanol/water (19: 1, v/v, 0.5 mL), and the suspension was stirred at room temperature for 3 days. The mixture was centrifuged, and the solid was collected and dried under vacuum at room temperature for 4 hours to obtain a crystal form I of a compound of formula (VI-1). The combination of high-performance liquid chromatography and ion chromatography (UPLC/IC) shows an acid-base molar ratio of 1.2, and the XRPD pattern is basically as shown in Figure 28.

### Example 12: Preparation of a crystal form J of a compound of formula (VII-1)

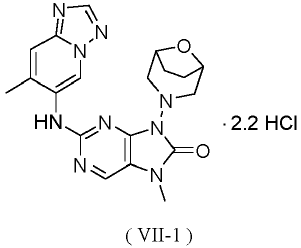

200 mg (1 *eq*) of the crystal form K of the compound of formula (VIII-1) was weighed and diluted with acetonitrile (2 mL), followed by the addition of 4 mol/L hydrochloric acid/methanol solution (0.5 mL), and after addition, the mixture was stirred at 25°C for 8 hours. The mixture was filtered, and the filter cake was dried under reduced pressure to obtain a crystal form J of a compound of formula (VII-1). The combination of high-performance liquid chromatography and ion chromatography (UPLC/IC) shows an acid-base molar ratio of 2.2, and the XRPD pattern is basically as shown in Figure 29.

### Example 13: Test on solid-state stability of the crystal form B of the compound of formula (II-1)

Based on the "Guideline for Stability Testing of Pharmaceutical Ingredients and Formulations" (Chinese Pharmacopoeia 2020 Edition, General Rule 9001), in order to evaluate the solid-state stability of the crystal form B of the compound of formula (II-1), the solid-state stability of the crystal form B was investigated in terms of the influencing factors (high temperature, high humidity, and light) and the conditions of 60°C/75% RH and 40°C/75% RH. The crystal form B was placed at high temperature (60 °C, open) and high humidity (25 °C/92.5% RH, open) for 10 days respectively, and then placed open under visible and UV light (samples of the shaded control group were placed at the same time and wrapped in tin foil) according to the ICH conditions (total illuminance up to 1,200,000 Lux hrs in visible and 200 W hrs/m² in UV), for 1, 2, and 3 months at 60°C/75% RH (open), and for 1, 2, and 3 months at 40°C/75% RH (open). XRPD tests were performed on all stability samples to detect changes in crystal form, and the results are shown in Table 16.

About 10 mg of this crystal form was accurately weighed and placed in a dry and clean glass vial, spread into a thin layer, and left open under the test conditions of the influence factors and the accelerated conditions. Samples placed under light conditions (visible light 1200000 Lux hrs, UV 200 W·hrs/m²) were in a clear glass vial with full exposure, and samples for XRPD test were placed separately.

After the sample was taken out at the time point, it was covered, sealed with a sealing film, and stored in a -20°C refrigerator. When preparing the sample, the sample was taken out of the refrigerator and warmed to room temperature, and dissolved with 25 mL of diluent (acetonitrile/water, 1:1, v/v) to obtain a solution with a concentration of approximately 0.5 mg/mL. The solution was analyzed using the liquid chromatography, and the results of the test were compared with the initial test results on day 0. The results of the test are shown in Table 16 below.

Preparation of standard solution on day 0: About 12.5 mg of this crystal form was weighed and placed in a 25 mL volumetric flask, dissolved with 50% acetonitrile, and diluted to the mark.

At the same time, HPLC tests were performed on all stability samples. The specific results are summarized in Table 16. The HPLC test instruments and analysis conditions are shown in Table 17.

**Table 16 Solid-state stability test results for the crystal form B of the compound of formula (II-1)**

| **Test conditions** | **Sampling conditions** | **Purity (area%)** | **Crystal form** |
|---|---|---|---|
| - | 0 days | 99.70 | Crystal form B |
| High temperature (60°C, open) | 10 days | 99.71 | Crystal form B |
| High humidity (25°C, relative humidity of 92.5%, open) | 10 days | 99.75 | Crystal form B |
| Visible light + UV (ICH conditions) | Total visible light illuminance reaches 1,200,000 Lux·hrs | 99.77 | Crystal form B |
| | Total UV illuminance reaches 200 W·hrs/m² | | |
| Shaded control group | It was placed with visible light + UV at the same time and wrapped with tin foil | 99.75 | Crystal form B |
| High temperature and high humidity (60°C, relative humidity of | One month | 99.76 | Crystal form B |
| | Two months | 99.73 | Crystal |
| 75%, open) | | | form B |
| | Three months | 99.73 | Crystal form B |
| High temperature and high humidity (40°C,relative humidity of 75%, open) | One month | 99.77 | Crystal form B |
| | Two months | 99.76 | Crystal form B |
| | Three months | 99.72 | Crystal form B |

**Table 17 HPLC instrument information and analysis methods**

| **Parameters** | **Set value** | | |
|---|---|---|---|
| Chromatography column | Waters Xbridge Shield RP 18, 150 × 4.6 mm, 3.5 µm | | |
| Mobile phase | A: pH5.5 10 mM ammonium acetate buffer; B: 100% acetonitrile | | |
| Gradient | Time (minutes) | Mobile phase A (%) | Mobile phase B (%) |
| | 0.00 | 90 | 10 |
| | 5.00 | 90 | 10 |
| | 45.00 | 55 | 45 |
| | 55.00 | 15 | 85 |
| | 60.00 | 15 | 85 |
| | 60.10 | 90 | 10 |
| | 70.00 | 90 | 10 |
| Detection time | 70 minutes | | |
| Flow rate | 0.5 mL/minutes | | |
| Injection volume | 5 µL | | |
| Detection wavelength | 220 nm | | |
| Column temperature | 35°C | | |
| Diluent | Acetonitrile/water (1:1, v:v) | | |

Conclusion: Crystal form B of the compound of formula (II-1) exhibits good chemical stability under all stability conditions (high temperature, high humidity, light exposure) without significant changes in purity and crystal form.

### Example 14: Study on hygroscopicity of the crystal form B of the compound of formula (II-1)

### Experimental materials:

SMS DVS intrinsic dynamic moisture sorption analyzer

### Experimental method:

The crystal form B of the compound of formula (II-1) (about 10 mg) was taken and placed in the DVS sample tray for testing.

### Experimental results:

The crystal form B of the compound of formula (II-1) has a DVS pattern shown in Figure 31, 2% > ΔW% ≥ 0.2%.

### Experimental Conclusion:

The crystal form B of the compound of formula (I) is slightly hygroscopic, with a hygroscopic weight gain of 2% > ΔW% ≥ 0.2% at 25°C and 80% RH.

### Bioassay data:

### Experimental example 1: DNA-dependent protein kinase (DNA-PK) inhibitory activity screening assay

This experiment was tested in the Eurofins

### Experimental materials and methods:

Human DNA-PK; Mg/ATP; GST-cMyc-p53; EDTA; Ser15 antibody; ATP: 10 µM.

### Experimental method (Eurofins Pharma Discovery Service):

DNA-PK(h) was incubated in assay buffer containing 50 nM GST-cMyc-p53 and Mg/ATP (according to the required concentration). The reaction was initiated by adding the Mg/ATP mixture. After incubation at room temperature for 30 minutes, the reaction was stopped by adding a stop solution containing EDTA. Finally, detection buffer (containing labeled anti-GST monoclonal antibody and europium-labeled anti-phospho Ser15 antibody against phosphorylated p53) was added. The plate was then read in time-resolved fluorescence mode, and the homogeneous time-resolved fluorescence (HTRF) signal was determined according to the formula HTRF = 10000 × (Em665nm/Em620nm).

Experimental results: The experimental results are shown in Table 18.

**Table 18 DNA-PK kinase activity test results**

| **Test sample** | **DNA-PK kinase inhibitory activity IC₅₀ (nM)** |
|---|---|
| Crystal form B of the compound of formula (II-1) | 0.6 |

Conclusion: The compound of the present disclosure has good inhibitory activity against DNA-PK.

### Example 2: Pharmacokinetic evaluation

### Experimental method

The test compound was mixed with 0.5% CMC-Na + 0.2% (V:V) Tween80 aqueous solution, vortexed, and sonicated to prepare a 3 mg/mL homogeneous suspension. SD male rats were selected, and orally administered with the candidate compound solution at a dose of 30 mg/kg. Whole blood was collected for a certain period of time and prepared into plasma. The drug concentration was analyzed by the LC-MS/MS method, and the pharmacokinetic parameters were calculated using Phoenix WinNonlin software (Pharsight, USA).

### Definition of each parameter:

Cₘₐₓ: highest plasma drug concentration after administration; Tₘₐₓ: time required to reach the peak concentration after administration; T_{1/2}: time required for the plasma drug concentration to decrease by half; Tₗₐₛₜ: time at the last detection point; AUC₀₋ₗₐₛₜ: area under the drug-time curve, which refers to the area enclosed by the plasma drug concentration curve and the time axis.

Test results: The experimental results are shown in Table 19.

**Table 19 PK test results of various crystal forms and salt forms of the compound in rat plasma**

| Parameters | Cₘₐₓ (nM) | Tₘₐₓ (h) | T_{1/2} (h) | Tₗₐₛₜ (h) | AUC₀₋ₗₐₛₜ (nM.h) |
|---|---|---|---|---|---|
| Crystal form K of the compound of formula (VIII-1) | 21728 | 1.50 | 2.42 | 32.0 | 127313 |
| Crystal form A of the compound of formula (I) | 14552 | 0.67 | 1.59 | ND | 83483 |
| Crystal form B of the compound of formula (II-1) | 11057 | 1.33 | 4.07 | ND | 87413 |

| | | | | | |
|---|---|---|---|---|---|
| Note: ND: Not determined (parameters could not be determined due to insufficient definition of terminated elimination phase) | | | | | |

Conclusion: The compound of the present disclosure exhibits longer half-life, higher drug exposure, and has better pharmacokinetic properties *in vivo.*

### Experimental Example 3: In vivo pharmacodynamic study

Experimental purpose: To study the *in vivo* pharmacodynamics of the test compound in the BALB/c nude mouse model with subcutaneous xenograft tumor of human small cell lung cancer NCI-H1703 cells.

Experimental animals: Experimental animals: female BALB/c nude mice, 6 to 8 weeks of age, weighing 18 to 22 grams; supplier: Shanghai Sippr-BK Lab.Animal Co.Ltd.

### Experimental methods and steps:

### (1) Cell culture

Human non-small cell lung cancer NCI-H1703 cells were cultured *in vitro* in RPMI1640 medium with 10% fetal bovine serum, 100 U/mL penicillin, and 100 µg/mL streptomycin, and cultured at 37°C in a 5% CO₂ incubator. Conventional digestion with trypsin-EDTA was performed twice a week for passaging. When the cell saturation was 80% to 90% and the number of cells reached the requirement, and the cells were collected, counted, and seeded.

### (2) Tumor cell seeding (tumor seeding)

0.2 mL (5×10⁶) NCI-H1703 cells (added with Matrigel, volume ratio of 1:1) were subcutaneously inoculated on the right back of each mouse, and the administration in groups began when the average tumor volume reached about 130 mm³.

### (3) Preparation of test sample

The compound of formula (I) was prepared into 3 mg/mL, 6 mg/mL, and 9 mg/mL suspension solutions with 98.5% water + 0.5% HPMC + 1% Tween 80.

### (4) Tumor measurement and experimental indicators

Tumor diameters were measured with a vernier caliper twice a week. The formula for calculating tumor volume is: V = 0.5*a* × *b*², wherein *a* and *b* represent the long and short diameters of the tumor, respectively. The relative tumor volume (RTV) was calculated based on the results of tumor measurement. The calculation formula is RTV = Vₜ/V₀, wherein V₀ is the tumor volume measured at the time of group administration (i.e., day 0), and Vt is the tumor volume at one measurement. Data of the same day was taken for T_{RTV} and C_{RTV}.

The tumor inhibitory effect of the compound was evaluated by TGI (%, reflecting the tumor growth inhibition rate) or relative tumor proliferation rate T/C (%):
T/C (%) = T_{RTV} / C_{RTV} × 100%, T_{RTV} represents the average RTV of the treatment group, and C_{RTV} represents the average RTV of the negative control group.
TGI (%) = [1 - (average tumor volume at the end of administration in a certain treatment group - average tumor volume at the beginning of administration in this treatment group) / (average tumor volume at the end of treatment in the vehicle control group - average tumor volume at the beginning of treatment in the vehicle control group)] × 100%.

After the experiment, the tumor weight (TW) was detected and T/C_{weight} (%) was calculated. The calculation formula is: T/C_{weight} (%) = TWₜᵣₑₐₜₘₑₙₜ / TW_{vehicle} × 100%. TWₜᵣₑₐₜₘₑₙₜ and TW_{vehicle} represent the tumor weights of the administration group and vehicle control group, respectively.

### (5) Statistical analysis

Statistical analysis was performed using SPSS software based on RTV data at the end of the experiment. The treatment group showed the best treatment effect on the 21st day after administration at the end of the experiment, so statistical analysis was performed based on this data to evaluate the differences between groups. T-test was used to analyze the comparison between two groups, and one-way ANOVA was used to analyze the comparisons between three or more groups. If the variance was homogeneous (F value had no significant difference), Tukey's method is used for analysis; if the variance was not homogeneous (F value had significant difference), and the Games-Howell method was used for testing. p < 0.05 was considered a significant difference.

### (6) Experimental conclusions and discussion

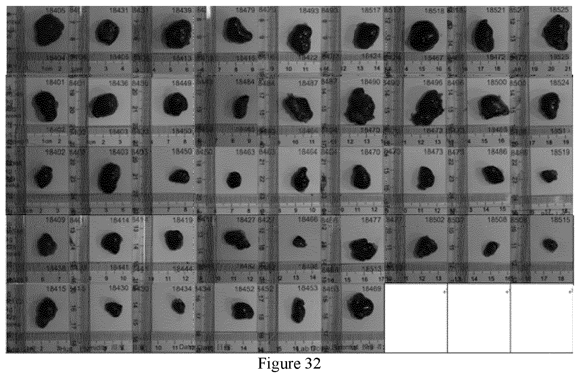

The experimental results are shown in Table 20 and Table 21, and the results of tumor photographs on day 21 are shown in Figure 32.

**Table 20 Tumor inhibitory effect of the compound of the present disclosure on human lung cancer NCI-H1703 xenograft tumor model**

| **Group** | **Tumor volume^{a} (mm³)** | **Tumor volume^{a} (mm³) (Day 21)** | **RTV (Day 21)** | **TGI (%) (Day 21)** | **T/C (%) (Day 21)** | ***p*** |
|---|---|---|---|---|---|---|
| | **(Day 0)** | | | | | |
| vehicle control | 131 ± 11 | 1200 ± 54 | 9.55 ± 0.69 | - | - | - |
| Compound of formula (I) (30 mg/kg) | 131 ± 10 | 979 ± 86 | 7.57 ± 0.53 | 20.6 | 79.3 | 0.202 |
| Compound of formula (I) (60 mg/kg) | 131 ± 10 | 427 ± 64 | 3.31 ± 0.51 | 72.3 | 34.7 | <0.001 |
| Compound of formula (I) (90 mg/kg) | 130 ± 10 | 351 ± 64 | 2.64 ± 0.45 | 79.4 | 27.7 | <0.001 |

| | | | | | | |
|---|---|---|---|---|---|---|
| Note: a. Mean ± SEM, n=9. | | | | | | |

**Table 21 Tumor weight and photographs of each experimental group**

| **Group** | **Tumor weight (g)^{a}** | **T/C_{weight}** | ***p* value** | **Corresponding photographs** |
|---|---|---|---|---|
| | **(Day 21)** | **(%)** | | |
| blank control | 1.296 ± 0.060 | - | - | First line |
| Compound of formula (I) (30 mg/kg) | 1.031 ± 0.095 | 79.6 | 0.301 | Second line |
| Compound of formula (I) (60 mg/kg) | 0.467 ± 0.078 | 36.0 | <0.001 | Third line |
| Compound of formula (I) (90 mg/kg) | 0.379 ± 0.069 | 29.2 | <0.001 | Fourth line |

| | | | | |
|---|---|---|---|---|
| **[0381]** Note: a. Mean ± SEM, n=9. | | | | |

Conclusion: In this experiment, the compound of formula (I) at the doses of 60 mg/kg and 90 mg/kg has a significant tumor inhibitory effect compared with the control group, and is dose-dependent. In this experiment, tumor-bearing mice show good tolerance to the compound of the present disclosure.

## Claims

1. A compound of formula (I), 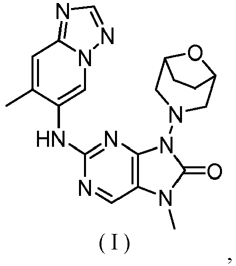 wherein a pharmaceutically acceptable salt of the compound is a methanesulfonate and a p-toluenesulfonate.

2. The methanesulfonate and the p-toluenesulfonate according to claim 1, wherein the methanesulfonate has a structure represented by formula (II), and the *p*-toluenesulfonate has a structure represented by formula (III), 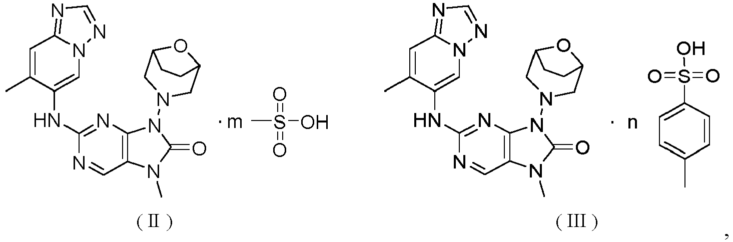 wherein
m is selected from 0.8, 0.9, 1.0, 1.1, 1.2, 1.5, 1.8, 1.9, 2.0, 2.1, 2.2;
n is selected from 0.8, 0.9, 1.0, 1.1, 1.2, 1.5, 1.8, 1.9, 2.0, 2.1, 2.2.

3. The methanesulfonate and the *p*-toluenesulfonate according to claim 2, wherein the compound of formula (II) is selected from a compound of formula (II-1), and the compound of formula (III) is selected from a compound of formula (III-1),

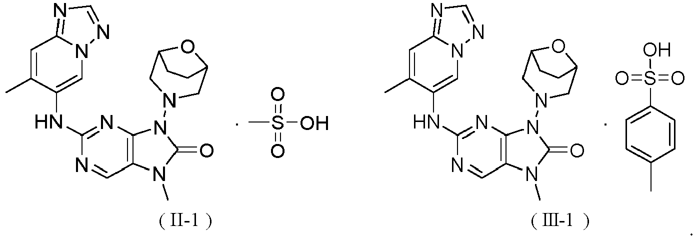

4. A crystal form B of a compound of formula (II-1), 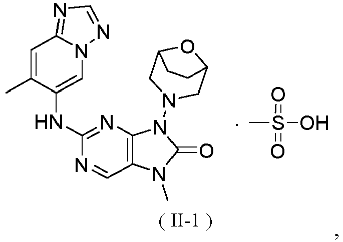 wherein the crystal form B has an X-ray powder diffraction pattern of Cu Kα radiation comprising characteristic diffraction peaks at the following 2θ angles: 7.02±0.20°, 16.56±0.20°, 21.25±0.20°.

5. The crystal form B according to claim 4, wherein the crystal form B has an X-ray powder diffraction pattern of Cu Kα radiation comprising characteristic diffraction peaks at the following 2θ angles: 7.02±0.20°, 10.66±0.20°, 13.98±0.20°, 16.56±0.20°, 21.25±0.20°.

6. The crystal form B according to claim 5, wherein the crystal form B has an X-ray powder diffraction pattern of Cu Kα radiation comprising characteristic diffraction peaks at the following 2θ angles: 7.02±0.20°, 9.29±0.20°, 10.66±0.20°, 13.98±0.20°, 16.56±0.20°, 21.25±0.20°, 29.98±0.20°.

7. The crystal form B according to claim 6, wherein the crystal form B has an XRPD pattern as shown in Figure 4.

8. The crystal form B according to any one of claims 4 to 7, wherein the crystal form B has a differential scanning calorimetry curve comprising an endothermic peak with an onset at 286.6°C±3°C.

9. The crystal form B according to claim 8, wherein the crystal form B has a DSC pattern as shown in Figure 5.

10. The crystal form B according to any one of claims 4 to 7, wherein the crystal form B has a thermogravimetric analysis curve with a weight loss of 0.58% at 200°C±3°C.

11. The crystal form B according to claim 10, wherein the crystal form B has a TGA pattern as shown in Figure 6.

12. A crystal form B of a compound of formula (II-1), 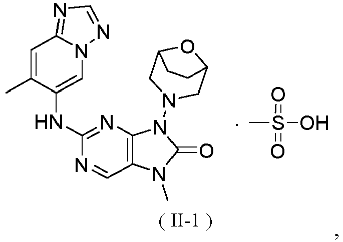
the crystal form B has a preparation method comprising the following steps:
(a) compound Z is added to solvent X;
(b) methanesulfonic acid is added dropwise at 25°C to 80°C, and the mixture is stirred at 25°C to 80°C for 3 to 16 hours after addition;
(c) solvent Y is added dropwise, and the mixture is stirred at 25°C to 80°C for 1 to 3 hours after addition;
(d) the mixture is cooled down to 15°C to 30°C;
(e) the mixture is filtered;
(g) the residue is dried under vacuum at 25°C to 60°C for 1 to 24 hours;
wherein,
the compound Z is selected from the compound of formula (I), the crystal form A of the compound of formula (I), and the crystal form K of the compound of formula (VIII-1);
the solvent X is selected from dimethyl sulfoxide, methanol, ethanol, acetonitrile, acetone, tetrahydrofuran and dichloromethane;
the solvent Y is absent, or the solvent Y is selected from methyl tert-butyl ether, ethyl acetate and n-heptane;
the methanesulfonic acid and the crystal form A of the compound of formula (I) has a molar ratio of 1.0:1 to 1.2:1.

13. A crystal form C of a compound of formula (III-1), 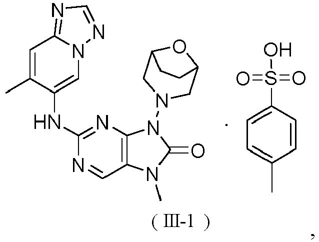 wherein the crystal form C has an X-ray powder diffraction pattern of Cu Kα radiation comprising characteristic diffraction peaks at the following 2θ angles: 5.96±0.20°, 15.16±0.20°, 17.83±0.20°.

14. The crystal form C according to claim 13, wherein the crystal form C has an X-ray powder diffraction pattern of Cu Kα radiation comprising characteristic diffraction peaks at the following 2θ angles: 5.96±0.20°, 7.05±0.20°, 10.23±0.20°, 15.16±0.20°, 17.83±0.20°, 22.11±0.20°, 23.19±0.20°.

15. The crystal form C according to claim 14, wherein the crystal form C has an X-ray powder diffraction pattern of Cu Kα radiation comprising characteristic diffraction peaks at the following 2θ angles: 5.96±0.20°, 7.05±0.20°, 10.23±0.20°, 11.89±0.20°, 15.16±0.20°, 17.83±0.20°, 19.09±0.20°, 19.96±0.20°, 22.11±0.20°, 23.19±0.20°.

16. The crystal form C according to claim 15, wherein the crystal form C has an X-ray powder diffraction pattern of Cu Kα radiation comprising characteristic diffraction peaks at the following 2θ angles: 5.96°, 7.05°, 8.08°, 10.23°, 11.89°, 12.30°, 12.99°, 14.01°, 15.16°, 16.67°, 17.54°, 17.83°, 18.75°, 19.09°, 19.47°, 19.96°, 20.67°, 21.6°, 22.11°, 23.19°, 23.83°, 25.18°, 26.69°, 28.32°, 29.90°, 30.48°, 32.39°, 35.02°.

17. The crystal form C according to claim 16, wherein the crystal form C has an XRPD pattern as shown in Figure 8.

18. The crystal form C according to any one of claims 13 to 17, wherein the crystal form C has a differential scanning calorimetry curve comprising an endothermic peak with an onset at 255.4±3°C.

19. The crystal form C according to claim 18, wherein the crystal form C has a DSC pattern as shown in Figure 9.

20. The crystal form C according to any one of claims 13 to 17, wherein the crystal form C has a thermogravimetric analysis curve with a weight loss of 2.90% at 150.0°C±3°C.

21. The crystal form C according to claim 20, wherein the crystal form C has a TGA pattern as shown in Figure 10.

22. A crystal form A of a compound of formula (I), 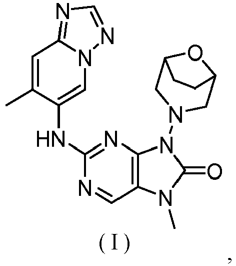 wherein the crystal form A has an X-ray powder diffraction pattern of Cu Kα radiation comprising characteristic diffraction peaks at the following 2θ angles: 9.26±0.20°, 19.47±0.20°, 22.69±0.20°.

23. The crystal form A according to claim 22, wherein the crystal form A has an X-ray powder diffraction pattern of Cu Kα radiation comprising characteristic diffraction peaks at the following 2θ angles: 9.26±0.20°, 11.50±0.20°, 13.52±0.20°, 17.03±0.20°, 18.75±0.20°, 19.47±0.20°, 22.69±0.20°, 27.74±0.20°.

24. The crystal form A according to claim 23, wherein the crystal form A has an X-ray powder diffraction pattern of Cu Kα radiation comprising characteristic diffraction peaks at the following 2θ angles: 5.77±0.20°, 9.26±0.20°, 11.50±0.20°, 13.52±0.20°, 17.03±0.20°, 17.50±0.20°, 18.75±0.20°, 19.47±0.20°, 22.69±0.20°, 23.84±0.20°, 24.42±0.20°, 26.76±0.20°, 27.74±0.20°.

25. The crystal form A according to claim 24, wherein the crystal form A has an X-ray powder diffraction pattern of Cu Kα radiation comprising characteristic diffraction peaks at the following 2θ angles: 5.77°, 9.26°, 11.50°, 13.52°, 14.50°, 15.16°, 15.60°, 17.03°, 17.28°, 17.50°, 18.47°, 18.75°, 19.27°, 19.47°, 22.69°, 23.84°, 24.42°, 26.76°, 27.74°, 28.43°, 29.77°, 30.40°, 32.08°.

26. The crystal form A according to claim 25, wherein the crystal form A has an XRPD pattern as shown in Figure 1.

27. The crystal form A according to any one of claims 22 to 26, wherein the crystal form A has a differential scanning calorimetry curve comprising an endothermic peak with an onset at 257.8°C±3°C.

28. The crystal form A according to claim 27, wherein the crystal form A has a DSC pattern as shown in Figure 2.

29. The crystal form A according to any one of claims 22 to 26, wherein the crystal form A has a thermogravimetric analysis curve with a weight loss of 1.39% at 230.0°C±3°C.

30. The crystal form A according to claim 29, wherein the crystal form A has a TGA pattern as shown in Figure 3.

31. A use of the methanesulfonate or p-toluenesulfonate according to any one of claims 1 to 3, the crystal form B according to any one of claims 4 to 11, the crystal form C according to any one of claims 13 to 21, the crystal form A according to any one of claims 22 to 30, or the crystal form prepared according to the method of claim 12 in the preparation of a DNA-PK inhibitor-related medicament.
